# EUROPÄISCHE PATENTANMELDUNG

(11) **EP 0 922 768 A2**
(43) Veröffentlichungstag der Anmeldung: **16.06.1999**
(21) Anmeldenummer: 98121471.1
(22) Anmeldetag: 11.11.1998
(51) Int. Cl.: C12N 15/85, C12N 15/63, A61K 31/70, A61K 48/00, A61K 38/17, C12N 5/10, C07K 14/47

(54) **Onkogen- oder virusgesteuerte Expressionssysteme**

(30) Priorität: 21.11.1997 DE 19751587
(71) Anmelder: Hoechst Marion Roussel Deutschland GmbH, 65929 Frankfurt am Main (DE)
(72) Erfinder: Müller, Rolf Prof.Dr., 35037 Marburg (DE); Sedlacek, Hans-Harald Prof.Dr., 35041 Marburg (DE)

(57) **Zusammenfassung**

Die Erfindung bezieht sich auf ein Nukleinsäurekonstrukt zur Expression eines Effektorgenes, wobei das Nukleinsäurekonstrukt einen Promotor I (Komponente a) enthält, der die Expression eines ebenfalls in Nukleinsäurekonstrukt enthaltenen Transkriptionsfaktorgenes (Komponente b) steuert und einen Promotor II (Komponente c) enthält, an den das Genprodukt des Transkriptionsfaktorgenes spezifisch bindet und das die Expression eines ebenfalls im Nukleinsäurekonstrukt enthaltenen Effektorgenes (Komponente d) steuert, dadurch gekennzeichnet, daß die Aktivität des Genprodukts des Transkriptionsfaktorgenes abhängig ist von einem oder mehreren zellulären Regulatorproteinen, die spezifisch an dieses Genprodukt binden und dessen Aktivität beeinflussen, eine isolierte Zelle, enthaltend besagtes Nukleinsäurekonstrukt, die Verwendung des besagten Konstrukts zur Herstellung eines Heilmittels zur Behandlung von Krankheiten, die Verwendung der besagten Zelle zu demselben Zweck und ein Verfahren zur Herstellung des Nukleinsäurekonstrukts.

## Beschreibung

Die vorliegende Erfindung bezieht sich auf ein Nukleinsäurekonstrukt zur Expression eines Effektorgenes, wobei das Nukleinsäurekonstrukt einen Promotor I (Komponente a) enthält, der die Expression eines ebenfalls im Nukleinsäurekonstrukt enthaltenen Transkriptionsfaktorgens (Komponente b) steuert und einen Promotor II (Komponente c) enthält, an den das Genprodukt des Transkriptionsfaktorgenes spezifisch bindet und das die Expression eines ebenfalls im Nukleinsäurekonstrukt enthaltenen Effektorgenes (Komponente d) steuert, dadurch gekennzeichnet ist, daß die Aktivität des Genprodukts des Transkriptionsfaktorgenes abhängig ist von einem oder mehreren zellulären Regulatorproteinen, die spezifisch an dieses Genprodukt binden und dessen Aktivität beeinflussen.

### I. Einleitung

Ein weithin unzureichend gelöstes Problem der Gentherapie ist die zellspezifische Kontrolle der Expression eines Effektorgenes besonders in erkrankten oder anderweitig veränderten Zellen. Die vorliegende Erfindung beinhaltet ein neues Verfahren dieser Kontrolle. Es basiert auf der Erkenntnis (Werness et al. Science 248, 76 (1990)), daß in entarteten Zellen Regulatorproteine auftreten, die derartig verändert oder vermindert sind, daß sie entweder nicht mehr an ihre zugehörigen Partnermoleküle binden und mit ihnen interagieren können, oder aber neue Bindungseigenschaffen mit ihren zugehörigen oder mit anderen Partnermolekülen gewinnen.

Das erfindungsgemäße Verfahren basiert desweiteren auf der Erkenntnis, daß beispielsweise das Retinoblastomprotein an die Aktivierungsdomäne des E2F-Transkriptionsfaktors binden und hierdurch dessen Aktivität inhibieren kann (Flemington et al., PNAS USA 90, 69 14 (1993)).

Gene für derartige Regulatorproteine wurden bereits für Expressionssysteme zur Suche nach Inhibitoren oder Stimulatoren dieser Regulatorproteine verwendet (z.B. WO95/19367, WO95/14777, WO97/04092).

Desweiteren offenbart wurden bereits Vektorsysteme, wobei ein erster Vektor ein Tumorsuppressorprotein exprimiert und ein zweiter Vektor ein Protein, welches an das Tumorsuppressorprotein bindet und es hierdurch inhibiert (WO 95/16771). Beide Vektoren werden in eine Zelle eingeführt. Durch die Kombination beider Vektoren können Vektoren, die ein Tumorsuppressorprotein kodieren, in der Zelle produziert werden, ohne daß die Zelle in ihrer Proliferation durch das Tumorsuppressorprotein inhibiert wird.

Zusätzlich werden in der WO 97/12970 Expressionssysteme offenbart, bei welchen die Expression eines ersten Genes durch einen ersten Promotor kontrolliert wird, dessen Funktion in Nicht-Tumorzellen supprimiert ist und die Expression eines zweiten Genes, dessen Expressionsprodukt die Expression des ersten Genes in Nicht-Tumorzellen hemmt, durch einen zweiten Promotor kontrolliert wird, der hochreguliert wird in Nicht-Tumorzellen.

Gegenstand der Erfindung ist nunmehr ein neues einfaches Expressionssystem, welches nur in Zellen, in denen derartige Regulatorproteine in verminderter oder veränderter Form vorkommen, aktiviert werden kann. Hierdurch wird die Transkription eines vom Expressionssystem kodierten Effektorgenes aktiviert. Das Expressionsprodukt des Effektorgenes hat alleine oder in Kombination mit einem weiteren pharmazeutischen Wirkstoff eine prophylaktische oder therapeutische Wirkung.

### II. Allgemeine Beschreibung der Erfindung

Das Expressionssystem entsprechend der Erfindung stellt ein Nukleinsäurekonstrukt dar, dessen Expression von Onkogenen oder Viren über die durch sie verursachten Veränderung oder Beeinflussung von Regulatorproteinen gesteuert wird und das im einfachsten Fall folgende Komponenten enthält:
a) mindestens eine Aktivierungssequenz (Promotoreinheit I)
b) mindestens ein Gen für einen Transkriptionsfaktor, wobei dessen Transkription durch die Komponente a) gesteuert wird
c) mindestens eine weitere Aktivierungssequenz (Promotoreinheit II), welche durch Bindung des Transkriptionsfaktors, kodiert von der Komponente b), die Expression der Komponente d) steuert
d) mindestens ein Effektorgen.

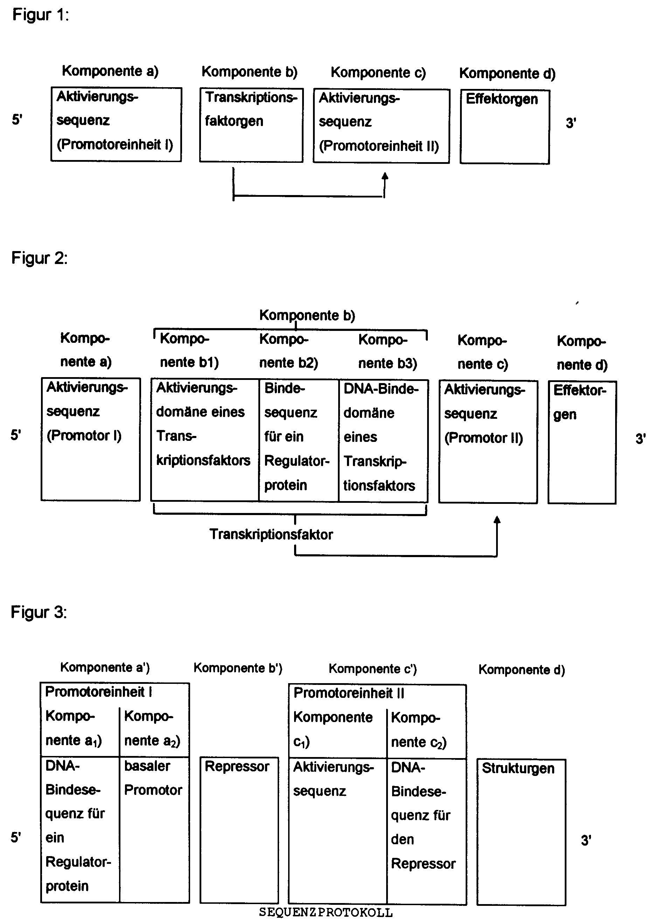

Die Anordnung der einzelnen Komponenten ist beispielhaft in Figur 1 wiedergegeben.

Entsprechend dieser Erfindung sind zwei besondere Ausführungsformen des Nukleinsäurekonstruktes zu unterscheiden:

### 1) Ausführungsform A)

Diese Ausführungsform ist durch folgende Eigenschaften der Komponenten gekennzeichnet:

### Komponente a)

- mindestens eine Aktivierungssequenz (Promotor Nr. I)

### Komponente b)

- mindestens ein Gen für einen Transkriptionsfaktor bestehend aus einem Fusionsprotein enthaltend
   - • Komponente b₁): mindestens eine Aktivierungsdomäne eines Transkriptionsfaktors
   - • Komponente b₂): mindestens eine Bindesequenz eines Bindeproteins für ein Regulatorprotein
   - • Komponente b₃): mindestens eine DNA-Bindedomäne eines Transkriptions faktors

### Komponente c)

- mindestens eine Aktivierungssequenz (Promotor Nr. II), welche durch Bindung des Transkriptionsfaktors, kodiert von der Komponente b) aktiviert wird

### Komponente d)

- mindestens ein Effektorgen.

Die Anordnung der einzelnen Komponenten ist beispielhaft in Figur 2 wiedergegeben. Voraussetzung für die erfindungsgemäße Funktionsfähigkeit des Expressionssystems ist, daß die Komponente b₂) derartig zwischen oder an die Komponenten b₁) und b₃) gefügt ist, daß die Bindung des Regulatorproteins an die Komponente b₂) die Funktionsfähigkeit der Aktivierungsdomäne (Komponente b₁) und/oder der DNA-Bindedomäne (Komponente b₃) inhibiert. Diese Inhibition führt in Normalzellen, d.h. bei normal funktionsfähigem Regulatorprotein, zu einer Inhibition der Expression des Effektorgenes. In einer entarteten oder infizierten Zelle, in welcher das Regulatorprotein entweder verändert oder komplexiert ist, so daß es nicht mehr mit dem zugehörigen Bindeprotein interagieren kann oder aber nicht mehr oder nur gering vorhanden ist, fehlt diese Inhibition, so daß der Transkriptionsfaktor (Komponente b) ungehindert die Aktivierungssequenz (Komponente c) aktivieren und damit die Transkription des Effektorgenes in die Wege leiten kann.
Die Transkription des Effektorgenes wird eingeleitet durch eine Aktivierung der Aktivierungssequenz [Komponente a)], welche eine Expression des Genes für den Transkriptionsfaktor [Komponente b)] zur Folge hat. Der Transkriptionstaktor [Komponente b)] wiederum bindet an die Aktivierungssequenz [Komponente c)], welche eine Expression des Effektorgenes [Komponente d)] induziert.

In einer besonderen Ausführungsform dieser Erfindung ist die Komponente a) gleich der Komponente c). In dieser besonderen Ausführungsform führt eine geringe Aktivierung der Aktivierungssequenz [Promotor I, Komponente a)] zu einer Expression des Transkriptionsfaktors [Komponente b)], welcher sowohl die Aktivierungssequenz [Promotor I, Komponente a)] als auch die Aktivierungssequenz [Promotor II, Komponente c)] akiviert und hierdurch die Expression sowohl des Effektorgenes [Komponente d)] induziert, als auch die Expression des Transkriptionsfaktors [Komponente b)] verstärkt, wodurch wiederum die Expression des Effektorgenes [Komponente d)] verstärkt wirkt.

### 2) Ausführungsform B)

Diese Ausführungsform ist durch folgende Eigenschaften der Komponenten gekennzeichnet:

### Komponente a')

- mindestens eine Aktivatorsequenz (Promotor I), welche enthält
   - • Komponente a₁):: mindestens eine DNA-Bindesequenz für ein Regulatorprotein und
   - • Komponente a₂):: mindestens einen basalen Promotor, wobei die Bindung des Regulatorproteins an die Komponente a1) die Komponente a₂) aktiviert

### Komponente b')

- mindestens einem Gen für einen als Repressor wirkenden Transkriptionsfaktor, wobei dessen Expression durch Komponente a') induziert wird

### Komponente c')

- mindestens eine Aktivierungssequenz (Promotor II), welche enthält
   - • Komponente c₁):: mindestens eine Aktivierungssequenz zur Induktion der Transkription der Komponente d) und
   - • Komponente c₂):: mindestens eine DNA-Sequenz zur Bindung des Repressors (Komponente b'), wobei diese Bindung die Aktivierung der Transkription des stromabwärtsgelegenen Effektorgenes (Komponene d) inhibiert

### Komponente d)

- ein Effektorgen.

Die Anordnung der Komponenten der Ausführungsform B) ist beispielhaft in Figur 3 wiedergegeben.

Voraussetzung für die erfindungsgemäße Funktionsweise des Expressionssystems gemäß Ausführungsform B) ist, daß in der Normalzelle die Bindung eines zellulären Regulatorproteins an die Komponente a') der Promotoreinheit I die Transkription des Repressorgenes (Komponente b') induziert und daß der exprimierte Repressor an die Komponente c₂) der Promotoreinheit II bindet und hierdurch die Aktivierung der Transkription des Strukturgenes (Komponente d) durch die Promotoreinheit II inhibiert.

In einer entarteten oder infizierten Zelle, in welcher das Regulatorprotein entweder verändert oder komplexiert ist, so daß es nicht mehr an die DNA-Bindesequenz (Komponente a₁) in der Promotoreinheit I binden kann oder aber nicht mehr oder nur gering vorhanden ist, erfolgt keine Expression des Genes für den Repressor und damit auch keine Inhibition der Expression des erfindungsgemäßen Nukleinsäurekonstruktes.

In diesen entarteten oder infizierten Zellen wird in der Ausführungsform B) des erfindinngsgemäßen Nukleinsäurekonstruktes die Transkription des Effektorgenes (Komponente d) in die Wege geleitet durch Aktivierung der Aktivierungssequenz (Komponente c₁) der Promotoreinheit II.

Dieses Expressionssystem kann erweitert werden

### in der Ausführungsform A) und B)

- durch die Aneinanderreihung mehrerer gleicher oder unterschiedlicher Sequenzen für Effektorgene [Komponente d), d'), d'')], welche jeweils miteinander durch gleiche oder unterschiedliche IRES-Sequenzen oder durch Aktivierungssequenzen [Komponenten c') und c'')] verbunden sind.

### In der Ausführungsform A)

- durch die Aneinanderreihung mehrerer gleicher oder unterschiedlicher Gene für Transkriptionsfaktoren [Komponenten b)], die jeweils miteinander durch gleiche oder unterschiedliche IRES-Sequenzen oder Aktivierungssequenzen [Komponente a)] oder Komponente c)] verbunden sind.

Bei einer Aneinanderreihung von Genen für unterschiedliche Transkriptionstaktoren sind die Aktivietungssequenzen so auszuwählen, daß sie Nukleotidsequenzen enthalten, an welche der Transkriptionsfaktor [Komponenten b)] binden kann.

Durch die erfindungsgemäßen Nukleinsäurekonstrukte kann ein Effektorgen [Komponente d)] je nach Wahl der Aktivierungssequenz [Komponenten a) oder c₁)] unspezifisch, zellspezifisch oder virusspezifisch oder unter bestimmten metabelischen Bedingungen oder auch Zellzyklus-spezifisch exprimiert werden. Bei dem Effektorgen handelt es sich um ein Gen, das seinerseits für einen pharmakologisch aktiven Wirkstoff oder aber für ein Enzym kodiert, welches eine inaktive Vorstufe eines Pharmakons in ein aktives Pharmakon spaltet. Beispielsweise kann das Effektorgen kann so ausgewählt sein, daß dieser Wirkstoff oder dieses Enzym als Fusionsprotein mit einem Liganden exprimiert wird und dieser Ligand an die Oberfläche von Zellen, zum Beispiel Endothel- oder Tumorzellen oder Leukozyten, bindet.

Die erfindungsgemäßen Nukleinsäurekonstrukte bestehen bevorzugterweise aus DNS. Unter dem Begriff "Nukleinsäurekonstrukte" werden künstliche Gebilde aus Nukleinsäure verstanden, die in den Zielzellen transkribiert werden können. Sie sind bevorzugt in einen Vektor eingefügt, wobei Plasmidvektoren oder virale Vektoren besonders bevorzugt sind.

Das Nukleinsäurekonstrukt, ggf. eingefügt in einen Vektor, wird einem Patienten zur Prophylaxe oder Therapie einer Erkrankung verabreicht. Die Verabreichung kann peroral, lokal oder per Injektion oder Infusion erfolgen.

Es können virale oder nicht-virale Vektoren benutzt werden. Virale Vektoren können zum Beispiel von RTV, AV, AAV oder HSV abgeleitet werden (Jolly, Cancer Gene Ther. 1, 51 (1994), oder man kann Plasmide verwenden, die mit kationischen Lipiden oder kationischen Polymeren komplexiert worden sind (Ledley, Human Gene Ther. 6, 1129, (1995). Solche Vektoren können in physiologischen Salzlösungen enthaltend 1% - 30% menschliches Albumin (vorzugsweise 5 %) gelöst sein.

1 x 10⁵ - 1 x 10¹⁰ PFU der viralen Vektoren (vorzugsweise 1 x 10⁸ PFU) oder 0,01mg - 50mg der Plasmide (vorzugsweise 1 mg) werden in 1 ml eines solchen Mediums suspendiert und beim Patienten appliziert. Die Applikation kann erfolgen durch Injektion (i.v., i.a., s.c., i.m.) in eine Körperhöhle (Pleura, Peritoneum, Subarachnoidal, in ein Gelenk) oder in ein Organ; oder es kann lokale Applikation erfolgen (intrabronchial, intranasal, dermal, intravaginal, in die Blase, in die Bindehaut.

Gegenstand der vorliegenden Erfindung sind auch Zellen von Säugern, die ein erfindungsgemäßes Nukleinsäurekonstrukt enthalten. In besonders bevorzugter Ausführungsform werden die Nukleinsäurekonstrukte in Zellinien eingebracht, die dann nach Transfektion als Träger des erfindungsgemäßen Expressionssystems zur Expression des Effektorgenes verwendet werden können. Derartige Zellen können zur Bereitstellung eines Heilmittels für Patienten benutzt werden. Alternativ können die Zellen oder Zellinien, wie z.B. Tumor-, Immun- oder Endothelzellen, in welche die erfindungsgemäßen Nukleinsäurekonstrukte eingebracht wurden, in Patienten lokal verabreicht oder parenteral zum Beispiel intravenös, intraarteriell in eine Körperhöhle, in ein Organ oder subkutan injiziert werden.

Eine bevorzugte Verwendung des erfindinngsgemäßen Nukleinsäurekonstruktes besteht somit in der Prophylaxe oder Behandlung einer Erkrankung, wobei die Erfindung die in vitro Einführung eines Nukleinsäurekonstruktes in eine Zielzelle, die unspezifische, virus- oder zielzellspezifische, metabolisch spezifische und/oder zellzyklusspezifische Expression des Heilmittels in der Zielzelle und die lokale oder parenterale Verabreichung der Zielzelle an den Patienten oder aber die lokale oder parenterale Verabreichung des Nukleinsäurekonstruktes an den Patienten zur in vivo Einführung eines Nukleinsäurekonstruktes in die Zielzelle umfaßt.

Beispiele sind Tumorzellen, die in vitro transduziert und intradermal oder subkutan injiziert werden zur Immunisierung von Patienten; oder CD4-positive T-Zellen, die so transduziert worden sind, daß sie einen neuen Rezeptor zur Umkehrung ihrer Zytotoxizität exprimieren, oder Muskelzellen, die so transduziert worden sind, daß sie Faktor IX exprimieren und die re-injiziert werden zur Behandlung einer defekten Faktor IX Produktion.

Die erfindungsgemäßen Nukleinsäurekonstrukte kommen in dieser Form nicht in der Natur vor, d.h. das Effektorgen für den Wirkstoff oder für ein Enzym oder für ein Ligand-Wirkstoff- oder Ligand-Enzym-Fusionsprotein ist nicht natürlicherweise kombiniert mit Nukleinsäuresequenzen, wie sie das erfindungsgemäße Nukleinsäurekonstrukt enthält.

Bevorzugte Effektorgene, welche in ein erfindungsgemäßes Expressionssystem eingebaut werden, kodieren für einen pharmakologisch aktiven Wirkstoff. Dieses sind Proteine und Glykoproteine, ausgewählt aus der Gruppe enthaltend Cytokine, Wachstumsfaktoren, Rezeptoren für Cytokine oder Wachstumsfaktoren, Antikörper oder Antikörperfragmente, antiproliferativ oder zytostatisch wirkende Proteine, apoptotisch oder antiapoptotisch wirkende Proteine, Tumorantigen, Angiogeneseinhibitoren, Thrombose-induzierende Proteine, Gerinnungshemmer, fibrinolytisch wirkende Proteine, Blutplasmaproteine, Komplement-aktivierende Proteine, Hüllsubstanzen von Viren und Bakterien, Hormone, kreislaufwirksame Peptide, Neuropeptide, Enzyme, Mediatoren, natürlicherweise vorkommende, nicht veränderte Regulatorproteine und Ribozyme oder auf die Genexpression inhibierend wirkende (antisense) Ribonukleotide.

Bevorzugterweise handelt es sich bei dem Transgen um ein Effektorgen, das für ein Ribozym kodiert, welches die mRNA inaktiviert, welche kodiert für ein Protein ausgewählt aus der Gruppe enthaltend Zellzykluskontrollproteine, inbesondere Cyclin A, Cyolin B, Cyolin D1, Cyolin E, E2F1-5, cdc2, cdc25C oder DP1 oder Virusproteine oder Cytokine oder Wachstumsfaktoren oder deren Rezeptoren.

In einer weiteren Ausführungsform kann das Effektorgen für ein Ligand-Wirkstofffusionsprotein kodieren, wobei der Ligand ein Antikörper, ein Antikörperfragment, ein Cytokin, ein Wachstumstaktor, ein Adhäsionsmolekül oder ein Peptidhormon sein kann und der Wirkstoff ein wie oben beschriebener pharmakologisch aktiver Wirkstoff oder ein Enzym. Beispielsweise kann das Effektorgen ein Ligand-Enzymfusionsprotein kodieren, wobei das Enzym eine Vorstufe eines Pharmakons in ein Pharmakon spaltet und der Ligand an eine Zelloberfläche bindet, bevorzugt an Endothelzellen oder Tumorzellen.

### III. Detaillierte Beschreibung der Besonderheiten der Ausführungsform A)

### 1) Die Komponente b)

### 1.1) Bindesequenz für ein Regulatorprotein [Komponente b₂)]

Zahlreiche zelluläre Bindeproteine für Regulatorproteine sind bereits beschrieben worden [Zwicker und Müller, Progress in Cell Cycle Res. 1: 91 (1995); Boulikas et al., Int. J. Oncol. 6: 271 (1995); Pawson, Nature 373: 573 (1995); Cotter, Leuk. Lymph. 18: 231 (1995); Hesketh, the Oncogene Facts Book Acad. Press, ISBN 0-12-344550-7 (1995); Miller and Sarver, Nature Med. 3: 389 (1997)].

Geeignet im Sinne der Erfindung sind inbesondere Bindeproteine oder deren Bindesequenzen für solche Regulatorproteine, welche in erkrankten Zellen nur gering experimiert sind, in ihrer Bindung an die Bindesequenz inhibiert sind, durch einen Überschuß der Bindesequenz nicht oder nur geringfügig in freier Form vorliegen oder in ihrer Funktion anderweitig, wie zum Beispiel durch Mutation, beeinträchtigt oder verändert sind.

Zu solchen Regulatorproteinen gehören beispielsweise die Proteine exprimiert von Tumorsuppressorgenen.

Eine die Erfindung nicht beschränkende Auswahl für derartige Regulatorproteine und ihre zugehörigen Bindeproteine und deren Bindesequenzen ist in folgenden Beispielen aufgeführt:

| Regulatorprotein | Komponente b₂) (zelluläres Bindeprotein mit Bindesequenz für das Regulatorprotein) |
|---|---|
| p53 | MDM-2 |
| pRb | • Transkriptionsfaktor E2F, -1, -2, -3 |
| | • Cyclin-D₁, D₂, -D₃, oder -C |
| | • Cyclin-A, -E |
| | • Transkriptionsfaktor PU.1 |
| | • Transkriptionsfaktor EIf-1 |
| p130 | • Transkriptionsfaktor E2F-5 |
| | • Cyclin A, - E |
| Max | • Myc |
| MAD | • Myc |
| VHL | • Elongin C, - B |
| cdk4 | p14, p15, p16, p18, p27, p57, p21 |
| MTS-1 (p16) | • cdk4 |
| WT-1 | • p53 |
| SMAD2 (MADR2) | • DPC4 |
| DPC-4 | • SMAD2 |
| β-catenin | • LEF-1 |
| LEF-1 | • β-catenin |

In einer besonderen Ausführungsform dieser Erfindung ist die Komponente b₂) eine Bindesequenz von einem nicht zelleigenen Bindeprotein für ein Regulatorprotein. Eine solche nicht-zelleigene Bindesequenz kann beispielsweise viralen, bakteriellen oder parasitären Ursprungs sein.

Die Verwendung einer derartigen nicht-zelleigenen Bindesequenz ermöglicht, daß in Normalzellen durch Bindung des zugehörigen Regulatorproteins an die Komponente b2) die Funktion der Komponente b) gehemmt ist. In infizierten Zellen wird jedoch durch die intrazelluläre Produktion des die Bindesequenz enthaltenden Bindeproteins durch den jeweiligen Infektionserreger das zugehörige Regulatorprotein weitgehend gebunden. Damit ist in diesen Zellen die Komponente b) frei und funktionsfähig.

In einer weiteren besonderen Ausführungsform dieser Erfindung ist die Komponente b₂) ein Antikörper oder einTeil eines Antikörpers mit Bindesequenzen (V_{H} und V_{L}) für ein Regulatorprotein.

Eine die Erfindung nicht beschränkende Auswahl nicht-zelleigener Bindesequenzen ist in folgenden Beispielen aufgeführt:

| Regulatorprotein | Komponente b₂) (virales Bindeprotein mit Bindesequenz für das Regulatorprotein) |
|---|---|
| p53 | • IE 84 von CMV (Speir et al., Science 265, 391 (1994) |
| | • E1B (55 Kd) von AV (Sarnow et al., Cell 28, 387 (1982); Liu et al., Cold Spring Harbor Symp. On Quantitative Biol. LIX, 215 (1995)) |
| | • EBNA-5 von EBV (Szekely et al., PNAs USA 90, 5455 (1993)) |
| | • BHFR1 von EBV (Theodorakis et al., Oncogene 12, 1707 (1996)) |
| | • E6 von HPV-16 oder -18 (Dyson et al., Science 243, 934 (1989); Howes et al., Genes Dev. 8, 1300 (1994)) |
| | • HBX protein von HBV (Wang et al., PNAS USA 91, 2230 (1994)) |
| | • T-Antigen von SV40 (Lane et al., Nature 278, 261 (1979); Linzer et al., Cell 17, 43 (1979)) |
| pRb | • E1A von AV (Nevins Science 258, 424 (1992)) |
| | • EBNA-2 von EBV |
| | • EBNA-1 oder -5 von EBV |
| | • E7 von HPV |
| | • T-Antigen von SV40 |
| p130 | • E1A von AV (Li et al., Genes Dev 7, 2366 (1993)) |
| CBF-1 (RBP-JK) | • EBNA-2 von EBV (Zimber-Strobl et al., EMBO J. 13, 4973 (1994)) |
| NF-Kappa B | • Tax von HIV (Suzuki et al., Oncogene 9, 3099 (1994)) |
| Lyn-tyrosinkinase | • LMP-1 von EBV |
| | • LMP-2A oder LMP-2B von EBV |
| bak | • E1B (16 Kd) von AV (Farrow et al., Nature 374, 731 (1995)) |
| bax | • E1B (19 kD) von Av (Han et al., Genes Dev. 10, 461 (1996)) |

| Regulatorprotein | Antikörper bzw. Antikörperfragmente mit Bindesequenz (V_{H}, V_{L}) für das Regulatorprotein |
|---|---|
| p53 | monoklonale Antikörper spezifisch für die nicht mutierte DNA-Bindedomäne (Legros et al., Oncogene 9, 2071 (1994); 9, 3689 (1994); Hupp et al., Cell 71, 875 (1992); Abarzna et al., Cancer Res. 55, 3490 (1995); Bonsing et al., Cytometry 28, 11 (1997); Thomas et al., J. Clin. Path. 50, 143 (1997); Jannot et al., BBRC 230, 242 (1997)) |
| pRb | • monoklonale Antikörper spezifisch für aktives (nicht phosphoryliertes) pRb (Hu et al., Mol. Cell Biol. 11, 5792 (1991)) |

Bei Wahl eines Antikörpers sind bevorzugt die epitopbindenen Teile des Antikörpers FV_{L} FV_{H} als Komponente b₂) einzusetzen, bei murinem Ursprung in humanisierter Form. Die Humanisierung erfolgt in der von Winter et al. (Nature 349, 293 (1991) und Hoogenbooms et al. (Rev. Tr. Transfus. Hemobiol. 36, 19 (1993)) dargestellten Weise. Die Antikörperfragmente werden entsprechend dem Stand der Technik hergestellt, beispielsweise in der von Winter et al., Nature 349, 293 (1991), Hoogenboom et al., Rev. Tr. Transfus. Hemobiol. 36, 19 (1993), Girol. Mol. Immunol. 28, 1379 (1991) oder Huston et al., Int. Rev. Immunol. 10, 195 (1993) beschriebenen Weise. Eine detaillierte Beschreibung der Herstellung von Antikörpern, Antikörperfragmenten und rekombinanten Antikörperfragmenten erfolgte in der Patentanmeldung DE 196 49 645.4.

Rekombinante Antikörperfragmente werden direkt aus existierenden Hybridomen hergestellt oder werden mit Hilfe der "phage display"-Technologie aus Bibliotheken muriner bzw. humaner Antikörperfragmente isoliert ('Winter et al., Annu. Rev. Immunol 12, 433 (1994)). Diese Antikörperfragmente werden dann auf genetischer Ebene direkt für die Kopplung mit den Komponenten b₁) und b₃) eingesetzt.

Zur Herstellung von rekombinanten Antikörperfragmenten aus Hybridomen wird die genetische Information, die für die antigenbindenden Domänen (V_{H}, V_{L}) der Antikörper kodiert, durch Isolierung der mRNA, die reverse Transkription der RNA in cDNA und die anschließende Amplifikation mittels Polymerasekettenreaktion und Oligoninkleotiden komplementär zu den 5'- bzw. 3'-Enden der variablen Fragmente gewonnen. Die so erhaltenen DNA-Fragmente kodierend für die V_{H}- und V_{L}-Fragmente werden dann in bakterielle Expressionsvektoren kloniert und so können z.B. Fv-Fragmente, einzelkettige Fv-Fragmente (scFv) oder Fab-Fragmente exprimiert werden.

Neue Antikörperfragmente können mittels der "phage display"-Technologie auch direkt aus Antikörpererbibliotheken (Immunbibliotheken, native Bibliotheken) murinen oder humanen Ursprungs isoliert werden. Beim "phage display" von Antikörperfragmenten werden die Gene antigenbindender Domänen als Fusionsgene mit dem Gen des Hüllproteins g3P filamentöser Bakteriophagen entweder in das Phagengenom oder in Phagemid-Vektoren in Form von scFv-Fragmentgenen oder als Fab-Fragmentgene kloniert. Antigen-bindende Phagen werden an antigenbeladenen Plastikgefäßen (panning), an antigenkonjugierten, paramagnetischen "beads" oder durch Bindung an Zelloberflächen selektioniert.

Immunbibliotheken werden hergestellt durch PCR-Amplifikation der Gene der variablen Antikörperfragmente aus B-Lymphozyten immunisierter Tiere oder Patienten. Dazu werden Kombinationen von Oligoninkleotiden, die spezifisch sind für murine oder humane Immunglobuline bzw. für die humanen Immunglobulin-Genfamilien verwendet.

Unter Verwendung nichtimmunisierter Spender als Quelle der Immunglobulingene lassen sich native Bibliotheken herstellen. Alternativ können Immunglobinlin-Keimbahngene zur Herstellung semisynthetischer Antikörparrepertoiras eingesetzt werden, wobei die Komplementarität-bestimmende Region 3 der variablen Fragmente durch PCR mit Hilfe degenerierter Primer ergänzt wird. Diese sogenannten "single pot"-Bibliotheken haben gegenüber Immunbibliotheken den Vorteil, daß Antikörperfragmente gegen eine Vielzahl von Antigenen aus einer einzigen Bibliothek isoliert werden können.

Die Affinität von Antikörperfragmenten kann mittels der "phage display"-Technologie weiter erhöht werden, wobei neue Bibliotheken von bereits existierenden Antikörperfragmenten durch zufällige, kodonbasierende oder gezielte Mutagenese durch "chain shuffling" einzelner Domänen mit Fragmenten aus naiven Repertoirss oder unter Zuhilfenahme von bakteriellen Mutatorstämmen hergestellt werden und durch Reselektion unter stringenten Bedingungen Antikörperfragmente mit verbesserten Eigenschaften isoliert werden. Zusätzlich können murine Antikörperfragmente durch stufenweisen Austausch einer der variablen Domänen gegen ein humanes Repertoire und anschließende Selektion mit dem ursprünglichen Antigen ("guided selection") humanisiert werden. Alternativ erfolgt die Humanisierung muriner Antikörper durch zielgerichteten Austausch der hypervariablen Regionen humaner Antikörper durch die korrespondierenden Regionen des originalen murinen Antikörpers.

### 1.2) Die Aktivierungsdomäne [Komponente b1)] und die DNA-Bindedomäne [Komponente b3)]

Im Sinne der Erfindung können alle verfügbaren Gene von Aktivierungsdomänen und DNA-Bindedomänen eines Transkriptionsfaktors für die Komponente b) verwendet werden. Beispiele hierfür, deren Beschreibung die Erfindung jedoch nicht einschränken soll, sind:
- Aktivierungdomänen [Komponente b₁)]
   mindestens eine Sequenz
   - der cDNA für die saure Transaktivierungsdomäne (TAD) von HSV1-VP16 (Aminosäuren 406 bis 488; Triezenberg et al., Genes Developm. 2: 718 (1988); Triezenberg, Curr. Opin. Gen. Developm. 5: 190 (1995) oder Aminosäuren 413 bis 490; Regier et al., Proc. Natl. Acad. Sci. USA 90, 883 (1993)) oder
   - der Aktivierungsdomäne von Oct-2 (Aminosäuren 438 bis 479; Tanaka et al., Mol. Cell. Biol. 14: 6046 (1994) oder Aminosäuren 3 bis 154; Das et al., Nature 374: 657 (1995)) oder
   - der Aktivierungsdomäne von SP1 (Aminosäuren 340 bis 485; Courey und Tijan, Cell 55, 887 (1988)) oder
   - der Aktivierungsdomäne von NFY (Aminosäuren 1 bis 233; Li et al., J. Biol. Chem. 267, 8984 (1992); van Hujisdinijnen et al., EMBO J. 9, 3119 (1990); Sinha et al., J. Biol. Chem. 92, 1624 (1995); Coustry et al. J. Biol. Chem. 270, 468 (1995)) oder
   - der Aktivierungsdomäne von ITF2 (Aminosäuren 2 bis 452; Seipel et al., EMBO J. 13, 4961, 1992)) oder
   - der Aktivierungsdomäne von c-Myc (Aminosäuren 1 bis 262; Eilers et al.) oder
   - derAktivierungsdomäne von CTF (Aminosäuren 399 bis 499; Mermod et al., Cell 58, 741 (1989); Das und Herr, Nature 374, 657 (1995))
- DNA-Bindedomänen [Komponente b₃)]
   mindestens eine Sequenz
   - der cDNA für die DNA-Bindedomäne des Gal4-Proteins (Aminosäuren 1 bis 147; Chasman und Kornberg, Mol. Cell. Biol. 10: 2916 (1990)) oder
   - des LexA-Proteins (Aminosäuren 1 bis 81; Kim et al., Science 255: 203 (1992) oder das ganze LexA-Protein (Aminosäuren 1 bis 202; Brent et al., Cell 43: 729 (1985)) oder
   - des lac-Repressor (lac I) Proteins (Brown et al., Cell 49: 603 (1987); Fuerst et al., PNAS USA 86: 2549 (1989)) oder
   - des Tetracyolin-Repressor(tet R)-Proteins (Gossen et al., PNAS USA 89; 5547 (1992); Dingermann et al., EMBO J. 11: 1487 (1992)) oder
   - des ZFHD1-Proteins (Pomerantz et al., Science 267: 93 (1995)).

Vorteilhaft im Sinne der Erfindung ist, an das 3' Ende der DNA-Bindedomäne ein nukleares Lokalisationssignal (NLS) anzufügen.

### 2) Die durch die Komponente b) aktivierbare Aktivierinngssequenz Promotoreinheit II [Komponente c)]

Die Auswahl dieser Aktivierungssequenz richtet sich nach der Wahl der DNA-Bindedomäne [Komponente b₃)] in dem Gen für einen Transkriptionsfaktor [Komponente b)].

Für die unter 1.2 aufgeführten Beispiele für die DNA-Bindedomänen bestehen wiederum beispielhaft folgende Möglichkeiten:

### 2.1) Möglichkeit A)

- eine Aktivierungssequenz
   mit mindestens einer Bindesequenz [Nukleotidsequenz: 5'-CGGACAACTGTT GACCG-3'] (SEQ ID NO.: 1) für das Gal4-Protein (Chasman und Kornberg, Mol. Cell Biol. 10, 2916 (1990)) und (an deren 3' Ende)
   - der basale Promotor von SV40 (Nukleotide 48 bis 5191; Tooze (ed), DNA Tumor Viruses (Cold Spring Harbor New York, New York; Cold Spring Harbor Laboratory) oder
   - der Promotor von c-fos (Das et al., Nature 374, 657 (1995)) oder
   - der U2 sn RNA-Promotor oder
   - der Promotor von HSV TK (Papavassiliou et al., J. Biol. Chem. 265, 9402 (1990); Park et al., Molec. Endocrinol. 7, 319 (1993)) engefügt ist.

### 2.2) Möglichkeit B)

- eine Aktivierungssequenz)
   - mit mindestens einer Bindesequenz [Nukleotidsequenz 5'-TACTGTATGTACA TACAGTA-3'] (SEQ ID NO.: 2) für das LexA Protein [LexA-Operator; Brent et al., Nature 612, 312 (1984)] und (en deren 3' Ende)
   - der besale Promotor von SV40 (Nukleotide 48 bis 5191; Tooze (ed), DNA Tumor Viruses (Cold Spring Harbor New York, New York; Cold Spring Harbor Laboratory) oder ein anderer Promotor (siehe Möglichkeit A) angefügt ist

### 2.3) Möglichkeit C)

- eine Aktivierungssequenz
   - mit mindestens einer Lac-Operator-Bindesequenz (Nukleotidsequenz: 5'-GAATTGTGAGCGCTCACAATTC-3') (SEQ ID NO.: 3) für das lac I Repressorprotein (Fuerst et al., PNAS USA 86, 2549 (1989); Simons et al., PNAS USA 81, 1624 (1984)) und (an deren 3' Ende)
   - der basale Promotor von SV40 (Nukleotide 48 bis 5191; Tooze (ed) DNA Tumor Viruses (Cold Spring Harbor New Yor, N.Y., Cold Spring Harbor Laboratory) oder einen anderen Promotor (siehe Möglichkeit A) angefügt ist.

### 2.4) Möglichkeit D)

- eine Aktivierungssequenz
   - mit mindestens einer Tetrazyklin-Operator-(tet O)-Bindesequenz (Nukleotidsequenz: 5'-TCGAGTTTACCAGTCCCTATCAGTGATAGAGAAAAG TGAAAG-3') (SEQ ID NO.: 4) für das Tetracyclin-Repressor-(tet R)-Protein und (an deren 3' Ende)
   - der basale Promotor von SV40 (Nukleotide 48 bis 5191; Tooze (ed.) DNA Tumor Viruses (Cold Spring Harbor New York, N.Y., Cold Spring Harbor Laboratory) oder ein anderer Promotor (siehe Möglichkeit A) angefügt ist

### 2.5) Möglichkeit E)

- eine Aktivierungssequenz
   - mit mindestens einer Bindesequenz [Nukleotidsequenz 5'-TAATGATGGCG3'] (SEQ ID NO.: 5) für das ZFHD-1 Protein (Pomerantz et al., Science 267, 93 (1995)) und (an dessen 3' Ende)
   - der basale Promotor von SV40 (Nukleotide 48 bis 5191; Tooze (ed.), DNA Tumor Viruses (Cold Spring Harbor New York, New York, Cold Spring Harbor Laboratory) oder einen anderen Promotor (siehe Möglichkeit A) angefügt ist.

### IV) Detaillierte Beschreibung der Besonderheiten der Ausführungsform B)

### 1) Die Aktivierungssequenz der Promotoreinheit I [Komponente a')]

### 1.1) Die DNA-Bindesequenz für ein Regulatorprotein [Komponente a1)]

Hierzu gehören die DNA-Bindesequenzen für Transkriptionsfaktoren, welche durch Mutation in ihrer DNA-Bindefähigkeit gehindert oder in der Zelle quantitativ vermehrt oder vermindert sind. Transkriptionsfaktoren und ihre Veränderungen wurden z.B. übersichtlich dargestellt von Nichols et al., Blood 80, 2953 (1992); Crepieux et al., Crit. Rev. Oncogen. 5, 615 (1994); LaThangue, TIBS 19, 108 (1994); Lipton, Nature Med. 3, 20 (1997)).

Beispielsweise gehört hierzu mindestens eine DNA-Bindesequenz
- für das p53 Protein
   [ATAATTGGGCAAGTCTAGGAA-3; (SEQ ID NO.: 6) Kern et al., Science 252, 1708 (1991), Cho et al., Science 265, 346 (1994) oder -(G/A)-(G/A)-(G/A)-C-(A/T)-(T/A)-G; Cho et al., Science 265, 348 (1994)]
- für das Wt-1 Protein
   (Wang et al., Oncogene 10, 415 (1995); Borel et al., Biochem. 35/37, 12070 (1996))
- für das NF-Kappa B Protein
   (Nukleotidsequenz 5'-GGGACTTTCC-3' (SEQ ID NO.: 7); Urban et al., Genes and Developm. 4, 1975 (1990); Roug et al., Virol. 189, 750 (1992)) oder HIV-LTR (Gimble et al., J. Virol. 62, 4104 (1988))
- für den E2F/DP-1 Komplex
   (mindestens eine Nukleotidsequenz 5'-TTTTCCCGCCAAAA (SEQ ID No.: 8); oder 5'-TTTTCCCGCCTTTTTT (SEQ ID NO.: 9) oder 5'-TTTTCCCGCGC TTTTTT) (DEQ ID NO.: 10) (Ouellete et al., Oncogene 7, 1075 (1992))
- für das Myc/Max Protein
   (mindestens eine Nukleotidsequenz von 5'-CACGTG-3') (Walhout et al., Nucl. Acids Res 25, 1493 (1997); Nozaki et al., J. Biochem. 121, 550 (1997)) oder von 5'-CATGTG-3' (Fisher et al., EMBO J. 12, 5075 (1993))

### 1.2) der basale Promotor [Komponente a₂)]

Hierzu gehören beispielsweise:
- der basale Promotor von SV40 (Nukleotide 48 bis 5191; Tooze (ed), DNA Tumor Viruses (Cold Spring Harbor New York, New York, Cold Spring Harbor Laboratory) oder
- der Promotor von c-fos (Das et al., Nature 374, 657 (1995)) oder
- der U2 sn RNA-Promotor oder
- der Promotor von HSV TK (Papavassiliou et al., J. Biol. Chem. 265, 9402 (1990); Park et al., Mol. Endocrin. 7, 319 (1993))

### 2) der Repressor [Komponente b')]

Hierzu gehören beispielsweise
- der lac-Repressor (Brown et al., Cell 49, 603 (1987); Fürst et al., PNAS USA 86, 2549 (1989)) oder
- der Tetracyclin-Repressor (Gossen et al., PNAS USA 89, 5549 (1992); Dingermann et al., EMBO J. 11, 1487 (1992))

### 3) die durch die Komponente b') beeinflußte Akivierungssequenz [Komponente c₁)]

Hierzu gehören beispielsweise alle nachfolgend im Abschnitt V) aufgeführten Aktivierungssequenzen.

### 4) die DNA-Bindesequenz für den Repressor [Komponente c₂)]

Hierzu gehören beispielsweise:
- mindestens eine Lac-Operator-Bindesequenz (Nukleotidsequenz: 5'-GAATTGTGAGCGCTCACAATTC-3') (SEQ ID NO.: 3) für das lac I Repressorprotein (Fürst et al., PNAS USA 86, 2549 (1989); Simons et al., PNAS USA 81, 1624 (1984)) oder
- mindestens eine Tetrazyklin-Operator-(tet o)-Bindesequenz (Nukleotidsequenz: 5'-TCGAGTTTACCACTCCCTATCAGTGATAGAGAAAAGTGAAAG-3') (SEQ ID NO.: 4) für das Tetrazyklin-Repressor-(tet R)-Protein.

### V) Die Aktivierungssequenz I [Komponente a) in Ausführungsform A) und Komponente c₁) in Ausführungsform B)]

Im Sinne der Erfindung sind als Aktivierungssequenzen Nukleotidsequenzen zu verwenden, welche nach Bindung von Transkriptionsfaktoren die Transkription eines am 3'-Ende benachbart gelegenen Genes aktivieren. Die Wahl der Aktivierungssequenz richtet sich nach der zu behandelnden Erkrankung und der zu transduzierenen Zielzelle. So kann die Aktivierungssequenz [Komponente a)] uneingeschränkt, zielzellspezifisch, unter bestimmten metabelischen Bedingungen, zellzyklusspezifisch oder virusspezifisch aktivierbar sein. Eine detaillierte Beschreibung dieser Promotorsequenzen erfolgte bereits in den Patentanmeldungen EP95930524.4, EP95931933.6, EP95931204.2, EP95931205.9 EP97101507.8, EP97102547.3, DE19639103.2 und DE19651443.6. Zu den auszuwählenden Promotorsequenzen gehören beispielsweise:

### 1) uneingeschränkt aktivierbare Promotoren und Aktivatorsequenzen, wie beispielsweise

- der Promotor der RNA-Polymerase III
- der Promotor der RNA-Polymerase II
- der CMV-Promotor und -Enhancer
- der SV40 Promotor

### 2) virale Promotor- und Aktivatorsequenzen, wie beispielsweise

- HBV
- HCV
- HSV
- HPV
- EBV
- HTLV
- HIV
Bei Verwendung des HIV-Promotors ist die gesamte LTR-Sequenz einschließlich der TAR-Sequenz [Position -453 bis -80, Rosen et al., Cell 41, 813 (1985)] als vitusspezifischer Promotor einzusetzen.

### 3) Metabolisch aktivierbare Promotor- und Enhancersequenzen,

wie beispielsweise der durch Hypoxie induzierbare Enhancer.

### 4) Zellzyklusspezifisch aktivierbare Promotoren

Solche sind beispielsweise der Promotor des cdc25C Gens, des Cyolin A Gens, des cdc2 Gens, des B-myb Gens, des DHFR-Gens, des E2F-1 Gens des cdc25B Genes, oder aber Bindesequenzen für während der Zellproliferdion auftretende oder aktivierte Transkriptionsfaktoren. Zu dieen Bindesequenzen gehören beispielsweise Bindesequuenzen für c-myc-Proteine. Zu diesen Bindesequenzen sind Monomere oder Multimere der als Myc E-Box bezeichneten Nukleotidsequenz [5'-GGAAGCAGACCACGTGGTCTGCTTCC-3' (SEQ ID NO.: 11); Blackwood and Eisenmann, Science 251: 1211 (1991)] zu zählen.

### 5) Tetrazyklin aktivierbare Promotoren,

wie beispielsweise der Tetrazyklin-Operator in Kombination mit einem entsprechenden Repressor.

### 6) Chimäre Promotoren

Ein chimärer Promotor stellt die Kombination einer stromaufwärts gelegenen zellspezifisch, metabolisch oder virusspezifisch aktivierbaren Aktivatorsequenz mit einem stromabwärts gelegenen Promotormodul dar, welches die Nukleotidsequenz CDE-CHR oder E2FBS-CHR enthält, an welche suppressive Proteine binden, die hierdurch die Aktivierung der stromaufwärts gelegenen Aktivatorsequenz in G₀ und G₁-Phase des Zellzyklus hemmen können (PCT/GB94/17366; Lucibello et al., EMBO J. 14, 12 (1994)).

### 7) Zellspezifisch aktivierbare Promotoren

Hierzu zählen bevorzugt Promotoren oder Aktivatorsequenzen aus Promotoren oder Enhancern von solchen Genen, die für Proteine bevorzugt gebildet in ausgewählten Zellen kodieren.

Zum Beispiel sind im Sinne der Erfindung in folgenden Zellen Promotoren für folgende Proteine bevorzugt zu verwenden:

### 7.1. Promotor- und Aktivatorsequenzen aktiviert in Endothelzellen

- Hirn-spezifischer, endothelialer Gluoose-1-Transporter
- Endoglin
- VEGF-Rezeptor-1 (flt-1)
- VEGF-Rezeptor-2 (flk-1, KDR)
- tie-1 oder tie-2
- B61-Rezeptor (Eck-Rezeptor)
- B61
- Endothelin, im speziellen Endothelin B oder Endothelin-1
- Endothelin-Rezeptoren, insbesondere der Endothelin B-Rezeptor
- Mannose-6-Phosphat-Rezeptoren
- von Willebrand Faktor
- IL-1α, IL-1β
- IL-1-Rezeptor
- Vascular Cell Adhesion Molecule (VCAM-1)
- synthetische Aktivatorsequenzen
   Als Alternative zu natürlichen endothelzellspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist [Lee et al., Biol. Chem. 266, 16188 (1991), Dormann et al., J. Biol. Chem. 267, 1279 (1992) und Wilson et al., Mol. Cell Biol. 10, 4854 (1990)].

### 7.2. Promotoren oder Aktivatorsequenzen, aktiviert in Zellen in Nachbarschaft aktivierter Endothelzellen

- VEGF
   Die genregulatorischen Sequenzen für das VEGF-Gen sind die 5' flankierende Region, die 3' flankierende Region, das c-Src-Gen oder das v-Src-Gen
- Steroid-Hormonrezeptoren und deren Promotorelemente (Truss und Beato, Endocr. Rev. 14, 459 (1993)), insbesondere der Maus-Mammatumor-virus-Promotor

### 7.3. Promotoren oder Aktivatorsequenzen aktiviert in Muskelzellen, insbesondere glatten Muskelzellen

- Tropomyosin
- α-Actin
- α-Myosin
- Rezeptor für PDGF
- Rezeptor für FGF
- MRF-4
- Phosphofruotokinase A
- Phosphoglyoeratemutase
- Troponin C
- Myogenin
- Rezeptoren für Endothelin A
- Desmin
- VEGF
   Die genregulatorischen Sequenzen für das VEGF-Gen sind bereits im Abschnitt "Promotoren aktiviert in Zellen in Nachbarschaft aktivierter Endothelzellen" aufgeführt worden (s.o.)
- "artifizielle" Promotoren

Faktoren der Helix-Loop-Helix (HLH)-Familie (MyoD, Myf-5, Myogenen, MRF4) sind als muskelspezifische Transkriptionsfaktoren beschrieben. Des weiteren gehören zu den muskelspezifischen Transkriptionsfaktoren das Zinkfingerprotein GATA-4.

Die HLH-Proteine sowie GATA-4 zeigen muskelspezifische Transkription nicht nur mit Promotoren von muskelspezifischen Genen, sondern auch im heterologen Kontext, so auch mit artifiziellen Promotoren. Derartige artifizielle Promotoren sind beispielsweise multiple Kopien der (DNA) Bindestelle für muskelspezifische HLH-Proteine wie der E-Box (Myo D) (z.B. 4x AGCAGGTGTTGGGAGGC) oder multiple Kopien der DNA Bindestelle für GATA-4 des α-Myosin-Heavy Chain Gens (z.B. 5'-GGCCGATGGGCA GATAGAGGGGGCCGATGGGCAGATAGAGG3') (SEQ ID NO.: 12)

### 7.4. Promotoren und Aktivatorsequenzen, aktiviert in Gliazellen

Hierzu zählen im besonderen die genreguldorischen Sequenzen bzw. Elemente aus Genen, die beispielsweise für folgende Proteine kodieren:
- das Schwannzell-spezifische Protein Petiaxin
- Glutaminsynthetase
- das Gliazell-spezifische Protein (Glial fibrillary acid protein = GFAP)
- das Gliazellprotein S100b
- IL-6 (CNTF)
- 5-HT-Rezeptoren
- TNFα
- IL-10
- Insulin-like Growth Factor Receptor I and II
- VEGF
   Die genregulatorischen Sequenzen für das VEGF-Gen sind bereits oben aufgeführt worden.

### 7.5. Promotoren und Aktivatorsequenzen aktiviert in blutbildenden Zellen

Zu solchen genregulatorischen Sequenzen gehören Promotorsequenzen für Gene eines Cytokins oder seines Rezeptors, die in blutbildenden Zellen oder in benachbarten Zellen, wie beispielsweise dem Stroma, exprimiert sind.

Hierzu gehören Promotorsequenzen für beispielsweise folgende Cytokine und ihre Rezeptoren:
- Stem Cell Factor-Receptor
- Stem Cell Factor
- IL-1α
- IL-1-Rezeptor
- IL-3
- IL-3-Rezeptor (α-subunit)
- IL-3-Rezeptor (β-subunit)
- IL-6
- IL-6-Rezeptor
- GM-CSF
- GM-CSF-Rezeptor (α-Kette)
- Interferon Regulatory Factor 1 (IRF-1)
   Der Promotor von IRF-1 wird durch IL-6 gleichermaßen aktiviert wie durch IFNγ oder IFNβ
- Erythropoietin
- Erythropoietin-Rezeptor.

### 7.6. Promotoren und Aktivatorsequenzen aktiviert in Lymphozyten und/oder Makrophagen

Hierzu gehören beispielsweise die Promotor- und Aktivatorsequenzen der Gene für Cytokine, Cytokinrezeptoren und Adhäsionsmoleküle und Rezeptoren für das Fc-Fragment von Antikörpern.

Hierzu gehören beispielsweise:
- IL-1-Rezeptor
- IL-1α
- IL-1β
- IL-2
- IL-2-Rezeptor
- IL-3
- IL-3-Rezeptor (α-subunit)
- IL-3-Rezeptor (β-subunit)
- IL-4
- IL-4-Rezeptor
- IL-5
- IL-6
- IL-6-Rezeptor
- Interferon Regulatory Factor 1 (IRF-1)
   (Der Promotor von IRF-1 wird durch IL-6 gleichermaßen aktiviert wie durch IFNγ oder IFNβ).
- IFNγ Responsive Promotor
- IL-7
- IL-8
- IL-10
- IL-11
- IFNγ
- GM-CSF
- GM-CSF-Rezeptor (α-Kette)
- IL-13
- LIF
- Makrophagen-Colony Stimulating Factor (M-CSF)-Rezeptor
- Typ I und II Makrophagen Scavenger Rezeptoren
- MAC-1 (Leukozflentunktionsantigen)
- LFA-1α (Leukozytenfunktionsantigen)
- p150,95 (Leukozflenfunktionsantigen)

### 7.7. Promotor- und Aktivatorsequenzen aktiviert in Synovialzellen

Hierzu gehören die Promotorsequenzen für Matrix-Metalloproteinasen (MMP), beispielsweise für:
- MMP-1 (interstitielle Kollagenase)
- MMP-3 (Stromelysin/Transin)

Hierzu gehören des weiteren die Promotorsequenzen für Tissue Inhibitors of Metalloproteinases (TIMP), beispielsweise
- TIMP-1
- TIMP-2
- TIMP-3

### 7.8. Promotoren und Aktivatorsequenzen aktiviert in Leukämiezellen

Hierzu gehören beispielsweise Promotoren für
- c-myc
- HSP-70
- bcl-1/cyclin D-1
- bcl-2
- IL-6
- IL-10
- TNFα, TNFβ
- HOX-11
- BCR-Abl
- E2A-PBX-1
- PML-RARA (Promyelocytic Leukemia - Retinoic Aoid Receptor)
- c-myc
- c-myc-Proteine binden an und aktivieren Multimere der als Myc E-Box bezeichneten Nukleotidsequenz (5'-GGAAGCAGACCAGCTGGTCT GCTTCC-3') (SEQ ID NO.: 11)

### 7.9. Promotoren oder Aktivatorsequenzen aktiviert in Tumorzellen

Als Promotor- oder Aktivdorsequenz ist eine genregulatorische Nukleotidsequenz vorgesehen, mit der Transkriptionsfaktoren, gebildet oder aktiv in Tumorzellen, interagieren.

Im Sinne dieser Erfindung zählen zu den bevorzugten Promotoren oder Aktivatorsequenzen genregulatorische Sequenzen bzw. Elemente aus Genen, die besonders in Krebszellen oder Sarkomzellen gebildete Proteine kodieren. So wird bei kleinzelligen Bronchialkarzinomen bevorzugt der Promotor des N-CAM-Proteins, bei Ovarialkarzinomen der Promotor des "Hepatitis growth faotor"-Rezeptors oder des L-Plastin und bei Pankreaskarzinomen der Promotor des L-Plastins oder des polymorphen epithelialen Mucins (PEM) verwendet.

### VI. Das Effektorgen [Komponente d)]

Im Sinne der Erfindung kodieren die Effektorgene [Komponente d)] für einen Wirkstoff zur Prophylaxe und/oder Therapie einer Erkrankung. Effektorgene und Promotorsequerzen sind im Hinblick auf die Art der Therapie der Erkrankung und unter Berücksichtigung der zu transduzierenden Zielzelle auszuwählen.

Beispielsweise sind bei folgenden Erkrankungen folgende Kombinationen von Promotorsequenzen und Effektorgenen zu wählen (eine detaillierte Beschreibung erfolgte bereits in den Patentanmeldungen EP95930524.4, EP95931933.6, EP95931204.2, EP95931205.9, EP97101507.8, DE19617851.7, DE19639103.2 und DE19651443.6, auf die Bezug genommen wird).

### 1) Therapie von Tumoren

### 1.1) Zielzellen:

- proliferierende Endothelzellen oder
- der Endothelzelle benachbarte Stromazellen und Muskelzellen oder
- Tumorzellen oder Leukämiezellen

### 1.2) Promotoren:

- endothelzellspezifisch und zellzyklusspezifisch oder
- zellunspezifisch oder muskelzellspezifisch und zellzyklusspeziliisch oder
- tumorzellspezifisch (solide Tumoren, Leukämien) und zellzyklusspezifisch

### 1.3) Effektorgene für Inhibitoren der Zellproliferation, zum Beispiel für

- das Retinoblastomprotein (pRb=p110) oder die verwandten p107 und p130 Proteine
   Das Retinoblastomprotein (pRb/p110) und die verwandten p107 und p130 Proteine werden durch Phosphorylierung inaktiviert. Bevorzugt sind solche Gene dieser Zellzyklusinhibitoren zu verwenden, welche Mutationen für die Inaktivietungsstellen der exprimierten Proteine aufweisen, ohne daß diese hierdurch in ihrer Funktion beeinträchtigt werden. Beispiele für diese Mutationen wurden beschrieben für das p110.
   In analoger Weise wird die DNA-Sequenz für das p107 Protein oder das p130 Protein mutiert.
- das p53 Protein
   Das Protein p53 wird in der Zelle inaktiviert entweder durch Bindung an spezielle Proteine, wie beispielsweise MDM2, oder durch Oligomerisierung des p53 über das dephosphorylierte C-terminale Serin. Bevorzugt wird somit eine DNA-Sequenz für ein p53 Protein verwendet, welches C-terminal verkürzt ist um das Serin 392
- das p21 (WAF-1)
- das p16 Protein
- andere cdk-Inhibitoren
- das GADD45 Protein
- das bak Protein
- ein Bindeprotein für ein Regulatorprotein (siehe II.1.)

### 1.4) Effektorgene für Gerinnung induzierende Faktoren und Angiogeneseinhibitoren, zum Beispiel:

- Plasminogenaktivatorinhibitor-1 (PAI-1)
- PAI-2
- PAI-3
- Angiostatin
- Interferone (IFNα, IFNβ oder IFNγ)
- Platelet factor 4
- TIMP-1
- TIMP-2
- TIMP-3
- Leukemia Inhibitory Factor (LIF)
- Tissue Factor (TF) und dessen gerinnungsaktive Fragmente

### 1.5) Effektorgene für zytostatische und zytotoxische Proteine, zum Beispiel für

- Perforin
- Granzym
- IL-2
- IL-4
- IL-12
- Interferone, wie beispielsweise IFN-α, IFNβ oder IFNγ
- TNF, wie TNFα oder TNFβ
- Oncostatin M
- Sphingomyelinase
- Magainin und Magainin-Derivate

### 1.6) Effektorgene für zytostatische oder zytotoxische Antikörper und für Fusionsproteine zwischen antigenbindenden Antikörperfragmenten mit zytostatischen, zytotoxischen oder entzündungserregenden Proteinen oder Enzymen.

- Zu den zytostatischen oder zytotoxischen Antikörpern gehören solche gerichtet gegen Membranstrukturen von Endothelzellen wie sie beispielsweise von Burrows et al. (Pharmac. Ther. 64, 155 (1994)), Hughes et al., (Cancer Res. 49, 6214 (1989)) und Maruyama et al., (PNAS USA 87, 5744 (1990)) beschrieben wurden. Insbesondere zählen hierzu Antikörper gegen die VEGF-Rezeptoren.
- Des weiteren gehören hierzu zytostatische oder zytotoxische Antikörper gerichtet gegen Membranstrukturen auf Tumorzellen. Derartige Antikörper wurden zum Beispiel von Sedlacek et al., Contrib. to Oncol. 32, Karger Verlag, München (1988) und Contrib. to Oncol. 43, Karger Vertag, München (1992) übersichtlich dargestellt. Weitere Beispiele stellen dar Antikörper gegen Sialyl Lewis; gegen Peptide auf Tumoren, welche von T-Zellen erkannt werden; gegen von Onkogenen exprimierte Proteine; gegen Ganglioside wie GD3, GD2, GM2, 9-0-acetyl GD3, Fucosyl GM1; gegen Blutgtuppenantigene und deren Vorläufer; gegen Antigene auf dem polymorphen epithelialen Mucin; gegen Antigene auf Heat Shock Proteinen
- Des weiteren gehören hierzu gehören Antikörper gerichtet gegen Membranstrukturen von Leukämiezellen. Eine große Anzahl derartiger monoklonaler Antikörper sind bereits für diagnostische und therapeutische Verfahren beschrieben worden (Übersichten bei Kristensen, Danish Medical Bulletin 41, 52 (1994); Schranz, Therapia Hungarica 38, 3 (1990); Drexler et al., Leuk. Res. 10, 279 (1986); Naeim, Dis. Markers 7, 1 (1989); Stickney et al., Curr.. Opin. Oncol. 4, 847 (1992); Drexler et al., Blut 57, 327 (1988); Freedman et al., Cancer Invest 9, 69 (1991)). Je nach Typ der Leukämie sind als Liganden beispielsweise monoklonalen Antikörper oder deren antigenbindende Antikörperfragmente gerichtet gegen folgende Membranantigene geeignet:

| Zellen | Membranantigen |
|---|---|
| AML | CD13 |
| | CD15 |
| | CD33 |
| | CAMAL |
| | Sialosyl-Le |
| B-CLL | CD5 |
| | CD1c |
| | CD23 |
| | Idiotypen und Isotypen der Membranimmunglobuline |

| Zellen | Membranantigen |
|---|---|
| T-CLL | CD33 |
| | M38 |
| | IL-2-Rezeptoren |
| | T-Zell-Rezeptoren |
| ALL | CALLA |
| | CD19 |
| | Non-Hodgkin Lymphoma |

- Die Humanisierung muriner Antikörper, die Herstellung und Optimierung der Gene für Fab und rek. Fv Fragmente erfolgt entsprechend der dem Fachmann bekannten Technik Die Fusion der rek. Fv-Fragmente mit Genen für zytostatische, zytotoxische oder entzündungserregende Proteinen oder Enzymen erfolgt gleichermaßen entsprechend dem dem Fachmann bekannten Stand der Technik.

### 1.7) Effektorgene für Fusionsproteine von Zielzell-bindenden Liganden mit zytostatischen und zytotoxischen Proteinen. Zu den Liganden gehören alle Substanzen, welche an Membranstrukturen oder Membranrezeptoren auf Endothelzellen binden. Beispielsweise gehören hierzu

- Cytokine wie beispielsweise IL-1 oder Wachstumsfaktoren oder deren Fragmente bzw. Teilsequenzen von ihnen, die an Rezeptoren exprimiert durch Endothelzellen binden, wie beispielsweise PDGF, bFGF, VEGF, TGF.
- Des weiteren gehören hierzu Adhäsionsmoleküle, welche an aktivierte und/oder proliferierende Endothelzellen binden. Hierzu gehören beispielsweise SLex, LFA-1, MAC-1, LECAM-1, VLA-4 oder Vitroneotin.
- Hierzu gehören des weiteren Substanzen, welche an Membranstrukturen oder Membranrezeptoren von Tumor- oder Leukämiezellen binden. Beispielsweise gehören hierzu Hormone oder Wachstumsfaktoren oder deren Fragmente bzw. Teilsequenzen von ihnen, die an Rezeptoren exprimiert durch Leukämiezellen oder Tumorzellen binden.
   Derartige Wachstumsfaktoren wurden bereits beschrieben (Übersichten bei Cross et al., Cell 64, 271 (1991), Aulitzky et al., Drugs 48, 667 (1994), Moore, Clin. Cancer Res. 1, 3 (1995), Van Kooten et al., Leuk. Lymph. 12, 27 (1993)).
- Die Fusion der Gene dieser an die Zielzelle bindenden Liganden mit zytostatischen, zytotoxischen oder entzündungserregenden Proteinen oder Enzymen erfolgt entsprechend dem Stand der Technik mit den dem Fachmann bekannten Methoden.

### 1.8.) Effektorgene für Induktoren von Entzündungen, zum Beispiel für

- IL-1
- IL-2
- RANTES (MCP-2)
- monocyte chemotaotic and activating factor (MCAF)
- IL-8
- maorophage inflammatory protein-1 (MIP-1α, -β)
- neutrophil activating protein-2 (NAP-2)
- IL-3
- IL-5
- human leukemia inhibitory factor (LIF)
- IL-7
- IL-11
- IL-13
- GM-CSF
- G-CSF
- M-CSF
- Cobra venom factor (CVF) oder Teilsequenzen vom CVF, welche dem menschlichen Komplementfaktor C3b funktionell entsprechen, d.h. welche an den Komplementfaktor B binden können und nach Spaltung durch den Faktor D eine C3 Konvertass darstellen
- der menschliche Komplementfaktor C3 oder seine Teilsequenz C3b
- Spaltprodukte des menschlichen Komplementfaktors C3, welche funktionell und strukturell dem CVF ähneln
- bakterielle Proteine, welche Komplement aktivieren oder Entzündungen auslösen, wie beispielsweise Porine von Salmonella typhi murium, "clumping" Faktoren von Staphylococcus aureus, Moduline besonders von gram-negativen Bakterien, "Major outer membrane protein" von Legionellen oder von Haemophilus influenzae Typ B oder von Klebsiellen oder M-Moleküle von Streptokokken Gruppe G.

### 1.9) Effektorgene für Enzyme für die Aktivierung von Vorstufen von Zytostatika, zum Beispiel für Enzyme, welche inaktive Vorsubstanzen (Prodrugs) in aktive Zytostatika (Drugs) spalten.

Derartige Substanzen und die jeweils zugehörigen Prodrugs und Drugs sind bereits von Deonarain et al. (Br. J. Cancer 70, 786 (1994)), Mullen, Pharmac. Ther. 63, 199 (1994)) und Harris et al. (Gene Ther. 1, 170 (1994)) übersichtlich beschrieben worden. Beispielsweise ist die DNA-Sequenz eines der folgenden Enzyme zu verwenden:
- Herpes Simplex Virus thymidinkinase
- Varizella Zoster Virus Thymidinkinase
- bakterielle Nitroreduktase
- bakterielle β-Gluouronidase
- pflanzliche β-Glucuronidase aus Secale cereale
- humane β-Glucuronidase
- humane Carboxy peptidase (CB) zum Beispiel CB-A der Mastzelle, CB-B des Pankreas oder bakterielle Carboxy peptidase
- bakterielle β-Laktamase
- bakterielle Cytosine deaminase
- humane Catalase bzw. Peroxidase
- Phosphatase, im besonderen humane alkalische Phosphatase, humane saure Prostataphosphatase oder Typ 5 saure Phosphatase
- Oxidase, im besonderen humane Lysyloxidase oder humane saure D-aminooxidase
- Peroxidase, im besonderen humane Gluthation Peroxidase, humane Eosinophilen Peroxidase oder humane Schilddrüsen Peroxidase
- Galaktosidase

### 2) Therapie von Autoimmunerkrankungen und Entzündungen

### 2.1) Zielzellen:

- proliferierende Endothelzellen oder
- Makrophagen und/oder Lymphozyten oder
- Synovialzellen

### 2.2) Promotoren:

- endothelzellspezifisch und Zellzyklusspezifisch oder
- makrophagen- und/oder lymphozytenspezifisch und/oder zellzyklusspezifisch oder
- synovialzellspezifisch und/oder Zellzyklusspezifisch

### 2.3) Effektorgene für die Therapie von Allergien, zum Beispiel für

- IFNβ
- IFNγ
- IL-10
- Antikörper bzw. Antikörperfragmente gegen IL-4
- lösliche IL-4-Rezeptoren
- IL-12
- TGFβ

### 2.4) Effektorgene für die Verhinderung der Abstoßung von transplantierten Organen, zum Beispiel für

- IL-10
- TGFβ
- lösliche IL-1-Rezeptoren
- lösliche IL-2-Rezeptoren
- IL-1-Rezeptorantagonisten
- lösliche IL-6-Rezeptoren
- immunsuppressive Antikörper oder deren V_{H} und V_{L} enthaltende Fragmente oder deren über einen Linker verbundene V_{H}- und V_{L}-Fragmente. Immunsuppressive Antikörper sind beispielsweise Antikörper spezifisch für den T-Zell-Rezeptor oder seinen CD3-Komplex, gegen CD4 oder CD8 des weiteren gegen den IL-2-Rezeptor, IL-1-Rezeptor oder IL-4-Rezeptor oder gegen die Adhäsionsmoleküle CD2, LFA-1, CD28 oder CD40

### 2.5) Effektorgene für die Therapie von Antikörper-mediierten Autoimmunerkrankungen, zum Beispiel für

- TGFβ
- IFNα
- IFNβ
- IFNγ
- IL-12
- lösliche IL-4-Rezeptoren
- lösliche IL-6-Rezeptoren
- immunsuppressive Antikörper oder dessen V_{H} und V_{L}-enthaltende Fragmente

### 2.6) Effektorgene für die Therapie von Zell-mediierten Autoimmunerkrankungen, zum Beispiel für

- IL-6
- IL-9
- IL-10
- IL-13
- TNFα oder TNFβ
- einen immunsuppressiven Antikörper oder dessen V_{H}- und V_{L}-enthaltende Fragmente

### 2.7) Effektorgene für Inhibitoren der Zellproliferation, zytostatische oder zytotoxische Proteine und Enzyme für die Aktivierung von Vorstufen von Zytostatika

Beispiele für Gene kodierend für derartige Proteine sind bereits im Abschnitt "Effektorgene für die Therapie von Tumoren" aufgeführt.

In gleicher Form wie dort bereits beschrieben, können im Sinne der Erfindung Effektorgene verwendet werden, welche für Fusionsproteine aus Antikörpern bzw. Fab oder rek. Fv-Fragmenten dieser Antikörper oder anderen Liganden spezifisch für die Zielzelle und den o.a. Cytokinen, Wachstumsfaktoren, Rezeptoren, zytostatischen oder zytotoxischen Proteinen und Enzymen kodieren.

### 2.8) Effektorgene für die Therapie der Arthritis

Im Sinne der Erfindung werden Effektorgene ausgewählt, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert.

Hierzu gehören zum Beispiel
- IL-1-Rezeptorantagonist (IL-1-RA);
   IL-1 -RA inhibiert die Bindung von IL-1α, β
- löslicher IL-1-Rezeptor;
   löslicher IL-1-Rezeptor bindet und inaktiviert IL-1
- IL-6
   IL-6 erhöht die Sekretion von TIMP und Superoxiden und vermindert die Sekretion von IL-1 und TNFα durch Synovialzellen und Chondrozyten
- löslicher TNF-Rezeptor
   löslicher TNF-Rezeptor bindet und inaktiviert TNF.
- IL-4
   IL-4 inhibiert die Bildung und Sekretion von IL-1, TNFα und MMP
- IL-10
   IL-10 inhibiert die Bildung und Sekretion von IL-1, TNF∝ und MMP und erhöht die Sekretion von TIMP
- Insulin-like growth factor (IGF-1)
   IGF-1 stimuliert die Synthese von extrazellulärer Matrix.
- TGFβ, im speziellen TGFβ1 und TGFβ2
   TGFβ stimuliert die Synthese von extrazellulärer Matrix.
- Superoxiddismutase
- TIMP, im speziellen TIMP-1, TIMP-2 oder TIMP-3

### 3) Therapie der mangelhaffen Bildung von Zellen des Blutes

### 3.1) Zielzellen:

- proliferierende, unreife Zellen des blutbildenden Systems oder
- Stromazellen benachbart den blutbildenden Zellen

### 3.2) Promotoren:

- spezifisch für blutbildende Zellen und/oder Zellzyklusspezifisch
- zellunspezifisch und zellzyklusspezifisch

### 3.3) Effektorgene für die Therapie der Anämie, zum Beispiel für

- Erythropoietin

### 3.4) Effektorgene für die Therapie der Leukopenie, zum Beispiel für

- G-CSF
- GM-CSF
- M-CSF

### 3.5) Effektorgene für die Therapie der Thrombozytopenie, zum Beispiel für

- IL-3
- Leukemia Inhibitory Factor (LIF)
- IL-11
- Thrombopoietin

### 4) Therapie von Schäden des Nervensystems

### 4.1) Zielzellen:

- Gliazellen oder
- proliferierende Endothelzellen

### 4.2) Promotoren:

- Gliazell-spezifisch und zellzyklusspezifisch oder
- Endothelzell-spezifisch und Zellzyklusspezifisch oder
- unspezifisch und Zellzyklusspezifisch

### 4.3) Effektorgene für neuronale Wachstumsfaktoren, zum Beispiel

- FGF
- Nerve growth factor (NGF)
   Brain-derived neurotrophic factor (BDNF)
- Neurotrophin-3 (NT-3)
- Neurotrophin-4 (NT-4)
- Ciliary neurotrophic factor (CNTF)

### 4.4) Effektorgene für Enzyme, zum Beispiel für

- Tyrosinhydroxylase
- Dopadecarboxylase

### 4.5) Effektorgene für Cytokine und deren Inhibitoren, welche die neurotoxische Wirkung von TNFα inhibieren oder neutralisieren, zum Beispiel für

- TGFβ
- lösliche TNF-Rezeptoren
- TNF-Rezeptoren neutralisieren TNFα
- IL-10
   IL-10 inhibiert die Bildung von IFNγ, TNFα, IL-2 und IL-4
- lösliche IL-1-Rezeptoren
- IL-1-Rezeptor I
- IL-1-Rezeptor II
- lösliche IL-1-Rezeptoren neutralisieren die Aktivität von IL-1
- IL-1-Rezeptor-Antagonist
- lösliche IL-6-Rezeptoren

### 5) Therapie von Störungen des Blutgerinnungs- und Blutkreislaufsystems

### 5.1) Zielzellen:

- Endothelzellen oder
- proliferierende Endothelzellen oder
- somatische Zellen in Nachbarschaft von Endothelzellen und glatte Muskelzellen oder
- Makrophagen

### 5.2) Promotoren:

- zellunspezifisch und zellzyklusspezifisch oder
- spezifisch für Endothelzellen, glatte Muskelzellen oder Makrophagen und zellzyklusspezifisch

### 5.3) Stukturgene für die Inhibition der Gerinnung oder für die Förderung der Fibrinolyse, zum Beispiel für

- Tissue Plasminogen Activator (tPA)
- Urokinase-type Plasminogen Activator (uPA)
- Hybride von tPA und uPA
- Protein C
- Hirudin
- Serin Proteinase Inhibitoren (Serpine), wie beispielsweise C-1S-Inhibitor, α1-Antitrypsin oder Antithrombin III
- Tissue Factor Pathway Inhibitor (TFPI)

### 5.4) Effektorgene für die Förderung der Gerinnung, zum Beispiel für

- F VIII
- F IX
- von Willebrand factor
- F XIII
- PAI-1
- PAI-2
- Tissue Factor and Fragmente hiervon

### 5.5) Effektorgene für Angiogenesefaktoren, zum Beispiel für

- VEGF
- FGF

### 5.6) Effektorgene für die Blutdrucksenkung, zum Beispiel für

- Kallikrein
- Endothelzell "nitric oxide synthase"

### 5.7) Effektorgene für die Inhibition der Proliferation von glatten Muskelzellen nach Verletzungen der Endothelschicht, zum Beispiel für

- ein antiproliferatives, zytostatisches oder zytotoxisches Protein oder
- ein Enzym zur Aufspaltung von Vorstufen von Zytostatika in Zytostatika wie bereits oben (unter Tumor) aufgeführt oder
- ein Fusionsprotein eines dieser Wirkstoffe mit einem Liganden, beispielsweise einem Antikörper oder Antikörperfragmenten spezifisch für Muskelzellen

### 5.8) Effektorgene für weitere Blutplasmaproteine, zum Beispiel für

- Albumin
- C1-Inaktivator
- Serum Cholinesterase
- Transferrin
- 1-Antritrypsin

### 6) Impfungen

### 6.1) Zielzellen:

- Muskelzellen oder
- Makrophagen und/oder Lymphozyten
- Endothelzellen

### 6.2) Promotoren:

- unspezifisch und zellzyklusspezifisch oder
- zielzellspezifisch und zellzyklusspezifisch

### 6.3) Effektorgene für die Prophylaxe von Infektionserkrankungen

Die Möglichkeiten, auf konventionellem Wege wirkungsvolle Impfstoffe herzustellen, sind beschränkt.
Demzufolge wurde die Technologie der DNA-Vakzine entwickelt. Diese DNA-Vakzinen werten jedoch Fragen zur Wirksamkeitsstärke auf.

Gemäß dieser Erfindung ist mit einer größeren Wirksamkeit der DNA-Vakzine zu rechnen.

Als Wirksubstanz ist die DNA eines vom Infektionserreger gebildeten Proteins auszuwählen, welches durch Auslösung einer Immunreaktion, d.h. durch Antikörperbindung und/oder durch zytotoxische T-Lymphozyten zur Neutralisierung und/oder zur Abtötung des Erregers führt. Derartige sogenannte Neutralisationsantigene werden als Impfantigene bereits angewandt (siehe Übersicht bei Ellis, Adv. Exp. Med. Biol. 327, 263 (1992)).

Bevorzugt im Sinne der Erfindung wird die DNA kodierend für Neutralisationsantigene folgender Erreger:
- Influenza A-Virus
- HIV
- Tollwut-Virus
- HSV (Herpes Simplex Virus)
- RSV (Respiratory Synoytial Virus)
- Parainfluenza-Virus
- Rotavirus
- VZV (Varizella Zoster Virus)
- CMV (Cytomegalo-Virus)
- Masern-Virus
- HPV (Humanes Papillomvirus)
- HBV (Hepatitis B-Virus)
- HCV (Hepatitis C-Virus)
- HDV (Hepatitis D-Virus)
- HEV (Hepatitis E-Virus)
- HAV (Hepatitis A-Virus)
- Vibrio Cholerae-Antigen
- Borrelia Burgdorferi
- Helicobacter pylori
- Malaria-Antigen
- Zu derartigen Wirksubstanzen im Sinne der Erfindung gehört jedoch auch die DNA eines Antiidiotyp-Antikörpers oder seiner Antigen-bindenden Fragmente, dessen Antigenbindungsstrukturen (die "complementary determining regions") Kopien der Protein- oder Kohlenhydratstruktur des Neutralisationsantigens des Infektionserregers darstellen.
   Derartige Antiidiotyp-Antikörper können besonders Kohlenhydratantigene bei bakteriellen Infektionserregern ersetzen.

Derartige antiidiotypische Antikörper und ihre Spaltprodukte wurden von Hawkins et al. (J. Immunother. 14, 273 (1993)) und Westerink und Apicella (Springer Seminars in Immunopathol. 15, 227 (1993)) übersichtlich beschrieben.

### 6.4) Effektorgene für "Tumorvakzinen"

- Hierzu gehören Antigene auf Tumorzellen. Derartige Antigene wurden zum Beispiel von Sedlacek et al., Contrib. to Oncol. 32, Karger Verlag, München (1988) und Contrib. to Oncol 43, Karger Verlag, München (1992) übersichtlich dargestellt.

Weitere Beispiele stellen die Gene für folgende Proteinantigene bzw. für die variable Region (V_{L}, V_{H}) von Antiidiotypantikörpern korrespondierend für folgende Nicht-Proteinantigene dar
- Ganglioside
- Sialyl Lewis
- Peptide auf Tumoren, welche von T-Zellen erkannt werden
- von Onkogenen exprimierte Proteine
- Blutgruppenantigene und deren Vorläufer
- Antigene auf tumorassoziiertem Mucin
- Antigene auf Heat Shock Proteinen

### 7) Die Therapie von chronischen Infektionserkrankungen

### 7.1) Zielzelle:

- Leberzelle
- Lymphozyt und/oder Makrophage
- Epithelzelle
- Endothelzelle

### 7.2) Promotoren:

- virusspezifisch oder zellspezifisch und zellzyklusspezifisch

### 7.3) Effektorgene, beispielsweise für

- ein Protein, welches zytostatische, apoptotische oder zytotoxische Wirkungen aufweist.
- ein Enzym, welches eine Vorstufe einer antiviralen oder zytotoxischen Substanz in die aktive Substanz spaltet.

### 7.4) Effektorgene für antivirale Proteine

- antiviral wirksame Cytokine und Wachstumsfaktoren. Hierzu zählen beispielsweise IFNα, IFNβ, IFN-γ, TNFβ, TNFα, IL-1 oder TGFβ
- Antikörper einer Spezifität, die das jeweilige Virus inaktiviert oder dessen V_{H} und V_{L} enthaltende Fragmente oder dessen über einen Linker verbundene V_{H} und V_{L} Fragmente herstellt wie bereits beschrieben.
   Antikörper gegen Virusantigen sind beispielsweise:
   anti HBV
   anti HCV
   anti HSV
   anti HPV
   anti HIV
   anti EBV
   anti HTLV
   anti Coxsackie Virus
   anti Hantaan Virus
- ein Rev bindendes Protein. Diese Proteine binden an die Rev-RNA und inhibieren Rev-abhängige posttranskriptionelle Stufen der Retrovirus-Genexpression. Beispiele für Rev-bindende Proteine sind:
   RBP9-27
   RBP1-8U
   RBP1-8D
   Pseudogene von RBP1-8
- Ribozyme, welche die mRNA von Genen für Zellzykluskontrollproteine oder die mRNA von Viren verdauen. Ribozyme katalytisch für HIV wurden beispielsweise von Christoffersen et al., J. Med. Chem. 38, 2033 (1995) übersichtlich beschrieben.

### 5) Effektorgene für antibakterielle Proteine

Zu den antibakteriellen Proteinen gehören beispielsweise Antikörper, die bakterielle Toxine neutralisieren oder Bakterien opsonieren. Beispielsweise gehören hierzu Antikörper gegen
Meningokokken C oder B
E.coli
Borrelia
Pseudomonas
Helicobacter pylori
Staphylococcus aureus

### VII. Kombination gleicher oder unterschiedlicher Effektorgene

Gegenstand der Erfindung ist des weiteren ein selbstverstärkendes ggf. pharmakologisch kontrollierbares Expressionssystem, in welchem eine Kombination der DNA-Sequenzen von zwei gleichen oder zwei unterschiedlichen Effektorgenen [Komponente c) und c')] vorliegt. Zur Expression beider DNA-Sequenzen ist eine weitere Promotorsequenz oder vorzugsweise die cDNA einer "internal ribosome entry site" (IRES) als regulatorisches Element zwischen beiden Effektorgenen geschaltet.

Eine IRES ermöglicht die Expression zweier über eine IRES miteinander verbundener DNA-Sequenzen.

Derartige IRES wurden beispielsweise von Montford und Smith (TIG 11, 179 (1995); Kaufman et al., Nucl. Acids Res. 19, 4485 (1991); Morgan et al., Nucl. Acids Res. 20, 1293 (1992); Dirks et al., Gene 128, 247 (1993); Pelletier und Sonenberg, Nature 334, 320 (1988) und Sugitomo et al., BioTechn. 12, 694 (1994)) beschrieben.

So kann beispielsweise die cDNA der IRES-Sequenz des Poliovirus (Position ≤ 140 bis ≥ 630 des 5' UTR verwendet werden.

Bevorzugt im Sinne der Erfindung sind über weitere Promotorsequenzen oder eine IRES-Sequenz Effektorgene zu verknüpfen, welche eine additive Wirkung aufweisen.
Im Sinne der Erfindung sind bevorzugte Kombinationen von Effektorgenen beispielsweise für

### 1) die Therapie von Tumoren

- gleiche oder unterschiedliche, zytostatische, apoptotische, zytotoxische oder entzündungserregende Proteine oder
- gleiche oder unterschiedliche Enzyme für die Spaltung der Vorstufe eines Zytostatikums

### 2) die Therapie von Autoimmunerkrankungen

- unterschiedliche Cytokine oder Rezeptoren mit synergistischer Wirkung zur Hemmung der zellulären und/oder humoralen Immunreaktion oder
- unterschiedliche oder gleiche TIMPs

### 3) die Therapie von mangelhafter Bildung von Zellen des Blutes

- unterschiedliche, hierarchisch aufeinanderfolgende Cytokine, wie beispielsweise IL-1, IL-3, IL-6 oder GM-CSF und Erythropoietin, G-CSF oder Thrombopoietin

### 4) die Therapie von Nervenzellschäden

- ein neuronaler Wachstumsfaktor und ein Cytokin oder der Inhibitor eines Cytokines

### 5) die Therapie von Störungen des Blutgerinnungs- und Blutkreislaufsystems

- ein Antithrombotikum und ein Fibrinolytikum (z.B. tPA oder uPA) oder
- ein zytostatisches, apoptotisches oder zytotoxisches Protein und ein Antithrombotikum oder ein Fibrinolytikum
   mehrere unterschiedliche, synergistisch wirkende Blutgerinnungsfaktoren, beispielsweise F VIII und vWF oder F VIII und F IX

### 6) Impfungen

- ein Antigen und ein immunstimulierendes Cytokin, wie beispielsweise IL-1α, IL-1β, IL-2, GM-CSF, IL-3 oder IL-4 Rezeptor
- unterschiedliche Antigene eines Infektionserregers oder unterschiedlicher Infektionserreger oder
- unterschiedliche Antigene eines Tumortyps oder unterschiedlicher Tumortypen

### 7) Therapie von viralen Infektionserkrankungen

- ein antivirales Protein und ein zytostatisches, apoptotisches oder zytotoxisches Protein

- Antikörper gegen unterschiedliche Oberflächenantigene eines Virus oder mehrere Viren

### 8) Therapie von bakteriellen Infektionserkrankungen

- Antikörper gegen unterschiedliche Oberflächenantigene und/oder Toxine eines Keimes

### VIII. Einfügung von Signalsequenzen und Transmembrandomänen

### 1) Verstärkung der Translation

Zur Verstärkung der Trsnslation kann am 3' Ende der Promotorsequenz und unmittelbar am 5' Ende des Startsignals (ATG) der Signal- bzw. Transmembransequenz die Nukleotidsequenz GCCACC oder GCCGCC eingefügt (Kozak, J. Cell Biol. 108, 299 (1989)) werden.

### 2) Erleichterung der Sekretion

Zur Erleichterung der Sekretion des Expressionsproduktes des Effektorgenes kann die ggf. in der DNA-Sequenz des Effektorgenes enthaltende homologe Signalsequenz ersetzt werden durch eine heterologe, die extrazelluläre Ausschleusung verbessernde Signalsequenz.

So kann beispielsweise die Signalsequenz für das Immunglobulin (DNA Position ≤ 63 bis ≥ 107; Riechmann et al., Nature 332, 323 (1988)) oder die Signalsequenz für das CEA (DNA-Position ≤ 33 bis ≥ 134; Schrewe et al., Mol. Cell Biol. 10, 2738 (1990); Berling et al., Cancer Res 50, 6534 (1990)) oder die Signalsequenz des humanen Respiratory Syncytial Virus Glycoproteins (cDNA der Aminosäuren ≤ 38 bis ≥ 50 oder 48 bis 65; Lichtenstein et al., J. Gen. Virol. 77, 109 (1996)) eingefügt werden.

### 3) Verankerung des Wirkstoffes

### 3.1) Zur Verankerung des Wirkstoffes in die Zellmembran der den Wirkstoff bildenden transduzierten Zelle kann alternativ oder zusätzlich zur Signalsequenz eine Sequenz für eine Transmembrandomäne eingeführt werden.

So kann beispielsweise die Transmembransequenz des menschlichen Makrophagenkolonie-stimulierenden Faktors (DNA-Position ≤ 1485 bis ≥ 1554; Cosman et al., Behring Inst. Mitt. 83, 15 (1988)) oder die DNA-Sequenz für die Signal- und Transmembranregion des menschlichen Respiratory Synoytial Virus (RSV)-Glykoproteins G (Aminosäuren 1 bis 63 oder deren Teilsequenzen, Aminosäuren 38 bis 63; Vijaya et al., Mol. Cell Biol. 8, 1709 (1988); Lichtenstein et al., J. Gen. Virol. 77, 109 (1996)) oder die DNA-Sequenz für die Signal- und Transmembranregion der Influenzavirus-Neuraminidase (Aminosäuren 7 bis 35 oder die Teilsequenz Aminosäuren 7 bis 27; Brown et al., J .Virol 62, 3824 (1988)) zwischen der Promotorsequenz und der Sequenz des Effektorgens eingefügt werden.

### 3.2) Zur Verankerung des Wirkstoffes in die Zellmembran der den Wirkstoff bildenden transduzierten Zellen kann jedoch auch die Nukleotidsequenz für einen Glykophospholipid-Anker eingefügt werden.

Die Einfügung eines Glykophospholipid-Ankers erfolgt am 3' Ende der Nukleotidsequenz für das Effektorgen und kann zusätzlich zur Einfügung einer Signalsequenz erfolgen.

Glykophospholipid-Anker sind beispielsweise für das CEA, für das N-CAM und für weitere Membranproteine, wie beispielsweise Thy-1, beschrieben worden (siehe Übersicht Ferguson et al., Ann. Rev. Biochem. 57, 285 (1988)).

### 3.3) Eine weitere Möglichkeit der Verankerung von Wirkstoffen an die Zellmembran entsprechend der vorliegenden Erfindung ist die Verwendung einer DNA-Sequenz für ein Ligand-Wirkstoff-Fusionsprotein. Die Spezifität des Liganden dieses Fusionsproteins ist gerichtet gegen eine Membranstruktur auf der Zellmembran der gewählten Zielzelle.

Zu den Liganden, welche an die Oberfläche von Zellen binden, gehören beispielsweise Antikörper oder Antikörperfragmente, gerichtet gegen Strukturen auf der Oberfläche von beispielsweise
- Endothelzellen. Insbesondere zählen hierzu Antikörper gegen die VEGF-Rezeptoren oder gegen Kinin-Rezeptoren
- oder von Muskelzellen, wie Antikörper gegen Actin oder Antikörper gegen Angiotensin II-Rezeptoren oder Antikörper gegen Rezeptoren für Wachstumsfaktoren, wie beispielsweise gegen EGF-Rezeptoren oder gegen PDGF-Rezeptoren oder gegen FGF-Rezeptoren oder Antikörper gegen Endothelin A-Rezeptoren
- Zu den Liganden gehören auch Antikörper oder deren Fragmente, welche gerichtet sind gegen tumorspezifische oder tumorassoziierte Antigene auf der Tumorzellmembran. Derartige Antikörper wurden bereits beschrieben.

Die murinen monoklonalen Antikörper sind bevorzugt in humanisierter Form einzusetzen. Fab und rek. Fv-Fragmente und deren Fusionsprodukte werden, wie bereits beschrieben, mit der dem Fachmann bekannten Technologie hergestellt.

Zu den Liganden gehören des weiteren alle Wirkstoffe, wie beispielsweise Cytokine oder Adhäsionsmoleküle, Wachstumsfaktoren oder deren Fragmente bzw. Teilsequenzen von ihnen, Mediatoren oder Peptidhormone, welche an Membranstrukturen oder Membranrezeptoren auf der jeweiligen ausgewählten Zelle binden. Beispielsweise gehören hierzu
- Liganden für Endothelzellen, wie IL-1, PDGF, bFGF, VEGF, TGGβ oder Kinin und Derivate oder Analoga von Kinn.
- Des weiteren gehören hierzu Adhäsionsmoleküle. Derartige Adhäsionsmoleküle, wie beispielsweise SLex, LFA-1, MAC-1, LeCAM-1, VLA-4 oder Vitronectin und Derivate oder Analoga von Vitronectin, wurden bereits für Endothelzellen beschrieben (Übersichten bei Augustin-Voss et al., J. Cell Biol. 119, 483 (1992); Pauli et al., Cancer Metast. Rev. 9, 175 (1990); Honn et al., Cancer Metast. Rev. 11, 353 (1992); Varner et al., Cell Adh. Commun 3, 367 (1995)).

Die Erfindung ist in nachfolgenden Beispielen näher beschrieben.

### IX. Beispiele zur näheren Beschreibung des Erfindungsgegenstandes

### 1) Herstellung eines onkogengesteuerten Expressionssystems.

Das erfindungsgemäße onkogengesteuerte Expressionssystem besteht aus folgenden, unterschiedlichen, stromabwärts aufeinanderfolgenden Nukleotidsequenzen:
- Komponente a)
   - dem Promotor des cdc25C-Gens (Nukleinsäuren -290 bis +121; Zwicker et al., EMBO J. 14, 4514 (1995); Zwicker et al., Nucl. Acids Res. 23, 3822 (1995))
- Komponente b)
   - dem nukleären Lokalisationssignal (NLS) von SV40 (SV40 large T, Aminosäuren 126 bis 132; PKKKRKV (SEQ ID NO.: 13); Dingwall et al., TIBS 16, 478 (1991))
   - der sauren Transaktivierungsdomäne (TAD) von HSV-1 VP16 (Aminosäuren 406 bis 488; Triezenberg et al., Genes Developm. 2, 718 (1988); Triezenberg, Curr. Opin. Gene Developm. 5, 190 (1995))
   - der RB-Bindesequenz des E2F-1 Proteins (Aminosäuren 409 bis 426 (LDYHFGLEEGEGIRDLFD) (SEQ ID NO.: 14); Flemington et al., PNAS USA 90, 6914 (1993); Helin et al., Cell 70, 337 (1992))
   - der cDNA für die DNA-Bindedomäne des Gal4-Proteins (Aminosäuren 1 bis 147; Chasman und Kornberg, Mol. Cell Bioil. 10, 2916 (1990))
- Komponente c)
   - 10x die Bindesequenz für die Gal4 DNA-Bindesequenz mit der Nukleotidsequenz 5'-CGGACAATGTTGACCG-3' ( SEQ ID NO.: 1) (Chasman und Kornberg, Mol. Cell Biol. 10, 2916 (1990)
   - dem basalen Promotor von SV40 (Nukleinsäuren 48 bis 5191; Toose (ed) DNA Tumor Viruses; Cold Spring Harbor, New York, New York, Cold Spring Harbor Laboratory)
- Komponente d)
   - der Sequenz GCCACC (Kodak, J. Cell Biol. 108, 229 (1989))
   - der cDNA für das Signalpeptid des Immunglobulins (Nukleotidsequenz 63 bis 107; Riechmann et al., Nature 332, 323 (1988))
   - der cDNA der β-Glucuronidäse (Nukleotidsequenz 93 bis 1982; Oshima et al, PNAS USA 84, 685 (1987)

Die Verknüpfung der einzelnen Bestandteile des Konstrukts erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzyme und DNA-Ligasen. Diese Enzyme sind käuflich zu erwerben.

Das so hergestellte Nukleotidkonstrukt wird in den pXP2 Plasmidvektor (Nordeen, BioTechniques 454 (1988)) einkloniert, der direkt oder in kolloidalen Dispersionssystemen für eine in vivo Applikation genutzt wird.

Mit dem beschriebenen Plasmid werden in Kultur gehaltene 3T3 Fibroblasten (RB positiv) und Osteosarkomzellen (SAOS-2, RB negativ) mit der dem Fachmann bekannten Methode (Lucibello et al., EMBO J. 132 (1995)) transfiziert und die Menge an β-Glucuronidase produziert von den Fibroblasten bzw. Osteosarkomzellen mit Hilfe von 4-Methylumbelliferyl-β-glucuconid als Substrat gemessen.

Zur Überprüfung der Zellzyklusspezifität werden die Osteosarkomzellen durch Entzug von Methionin über 48 Stunden in G₀/G₁ synchronisiert. Der DNA-Gehalt der Zellen wird nach Anfärbung mit Hoechst 33258 im Fluoreszenzaktivierungs-Cell Sorter bestimmt (Lucibello et al., EMBO J. 132 (1995)).

Folgende Ergebnisse werden erzielt:

In transfizierten Fibroblasten (RB positiv) kann keine Zunahme der β-Glucuronidase im Vergleich zu nichttransfizierten Fibroblasten ermittelt werden.

Transfizierte Osteosarkomzellen (RB negativ) exprimieren deutlich mehr β-Glucuronidase als nichttransfizierte Osteosarkomzellen.

Proliferierende Osteosarkomzellen (DNA > 2S; S = einfacher Chromosomensatz) sekretieren deutlich mehr β-Glucuronidase als in G₀/G₁ synchronisierte Osteosarkomzellen (DNA = 2S).

Somit führt das beschriebene Expressionssystem zu einer RB-abhängigen Expression des Strukturgenes β-Glucuronidase, die abhängig von der Wahl der Promotorsequenz z.B. zellzyklusabhängig reguliert werden kann.

### 2) Herstellung eines virusgesteuerten Expressionssystems

Das erfindungsgemäße virusgesteuerte Expressionssystem besteht aus folgenden, unterschiedlichen, stromabwärts aufeinanderfolgenden Nukleotidsequenzen:
- Komponente a)
   - der Promotor des cdc25C-Gens (Nukleinsäuren -290 bis +121; Zwicker et al., EMBO J. 14, 4514 (1995); Zwicker et al., Nucl. Acids Res. 23, 3822 (1995))
- Komponente b)
   - dem nukleären Lokalisationssignal (NLS) von SV40 (SV40 large T, Aminosäuren 126 bis 132; PKKKRKV (SEQ ID NO.: 13); Dingwall et al., TIBS 16, 478 (1991))
   - der sauren Transaktivierungsdomäne (TAD) von HSV-1 VP16 (Aminosäuren 406 bis 488; Triezenberg et al., Genes Developm. 2, 718 (1988); Triezenberg, Curr. Opin. Gene Developm. 5, 190 (1995))
   - dem E6-Protein des HPV-18 Virus (Nukleotidsequenz 100 bis 578; Roggenbuck et al., J. Virol 65, 5068 (1991))
   - der cDNA für die DNA-Bindedomäne des Gal4-Proteins (Aminosäuren 1 bis 147; Chasmen und Kornberg, Mol. Cell Biol. 10, 2916 (1990))
- Komponente c)
   - 10x die Bindesequenz für die Gal4 DNA-Bindesequenz mit der Nukleotidsequenz 5'-CGGACAATGTTGACCG-3' (SEQ ID NO.: 1) (Chasman und Kornberg, Mol. Cell Biol. 10, 2916 (1990)
   - dem basalen Promotor von SV40 (Nukleinsäuren 48 bis 5191; Toose (ed) DNA Tumor Viruses; Cold Spring Harbor, New York, New York, Cold Spring Harbor Laboratory)
- Komponente d)
   - der Sequenz GCCACC (Kodak, J. Cell Biol. 108, 229 (1989))
   - der cDNA für des Signalpeptid des Immunglobulins (Nukleotidsequenz 63 bis 107; Riechmann et al., Nature 332, 323 (1988))
   - der cDNA der β-Glucuronidase (Nukleotidsequenz 93 bis 1982; Oshima et al, PNAS USA 84, 685 (1987)

Die Verknüpfung der einzelnen Bestandteile des Konstruktes erfolgt über geeignete Restriktionstellen, die über PCR Amplifikation an den Termini der verschiedenen Elemente mitgeführt werden. Die Verknüpfung erfolgt mit Hilfe von dem Fachmann bekannten, für die Restriktionsstellen spezifschen Enzymen und DNA-Ligasen. Diese Enzyme sind käuflich zu erwerben.
Das so hergestellte Nukleotidkonstrukt wird in einem pUC18/19 Plasmidvektor einkloniert, der direkt oder in kolloidalen Dispersionssystemen für eine in vivo Applikation genutzt wird.

Mit dem beschriebenen Plasmid werden in Kultur gehaltene menschliche Fibroblasten (Wi-38, E6/E7 negativ) und Cervixkarzinomzellen (HeLa, HPV-18-E6/E7 positiv) mit der dem Fachmann bekannten Methode (Lucibello et al., EMBO J. 132 (1995)) transfiziert und die Menge an β-Glucuronidase, produziert von diesen Zellen, mit Hilfe von 4-Methylumbelliferyl-β-glucuronid als Substrat gemessen.

Zur Überprüfung der Zellzyklusspezifität werden HeLa-Zellen durch Entzug von Methionin über 48 Stunden in G₀/G₁ synchronisiert. Der DNA-Gehalt der Zellen wird nach Anfärbung mit Hoechst 33258 im Fluoreszenzaktivierungs-Cell Sorter bestimmt (Lucibello et al., EMBO J. 132 (1995)).

Folgende Ergebnisse werden erzielt:

In transfizierten Fibroblasten kann keine Zunahme der β-Glucuronidase im Vergleich zu nichttransfizierten Fibroblasten ermittelt werden.
Transfizierte HeLa-Zellen exprimieren deutlich mehr β-Glucuronidase als nichttransfizierte HeLa-Zellen.

Proliferierende HeLa-Zellen (DNA > 2S; S = einfacher Chromosomensatz) sekretieren deutlich mehr β-Glucuronidase als in G₀/G₁ synchronisierte HeLa-Zellen (DNA = 2S).

Somit führt das beschriebene Expressionssystem zu einer virusspezifischen (HPV18) Expression des Strukturgenes β-Glucuronidase, die abhängig von der Wahl des Promotorsequenz z. B. zellzyklusabhängig reguliert werden kann.

Ein Wirkstoff gemäß den Beispielen 1 und 2 ermöglicht nach lokaler Applikation beispielsweise an den Ort des Tumors oder nach intrakranieller bzw. subarachnoidaler Gabe oder systemischer, bevorzugt intravenöser oder intraarterieller Gabe, daß vorwiegend, wenn nicht ausschließlich, nur solche Zellen β-Glucuronidase ausscheiden, welche ein mutiertes Onkogen oder eine Virusinfektion aufweisen. Diese β-Glucuronidase spaltet ein nunmehr injiziertes, gut verträgliches Doxorubicin-β-glucuronid (Jacquesy et al., EP 0 511 917 A1) in das zytostatisch wirkende Doxorubicin. Dieses hemmt die Endothelzellproliferation und wirkt zytostatisch auf diese Zellen wie auch auf benachbarte Tumorzellen. Hierdurch kommt es zur Hemmung des Tumorwachstums.

### Figurenlegende

Figur 1: Art und Anordnung der allgemeinen Komponenten eines erfindungsgemäßen Nukleinsäurekonstrukts.
Figur 2: Schematische Darstellung der Anordnung der allgemeinen Komponenten eines erfindungsgemäßen Nukleinsäurekonstrukts gemäß Ausführungsform A der Erfindung.
Figur 3: Schematische Darstellung der Anordnung der allgemeinen Komponenten eines erfindungsgemäßen Nukleinsäurekonstrukts gemäß Ausführungsform B der Erfindung.

## Patentansprüche

1. Nukleinsäurekonstrukt zur Expression eines Effektorgenes, wobei das Nukleinsäurekonstrukt einen Promotor I (Komponente a) enthält, der die Expression eines ebenfalls im Nukleinsäurekonstrukt enthaltenen Transkriptionsfaktorgenes (Komponente b) steuert und einen Promotor II (Komponente c) enthält, an den das Genprodukt des Transkriptionsfaktorgenes spezifisch bindet und das die Expression eines ebenfalls im Nukleinsäurekonstrukt enthaltenen Effektorgenes (Komponente d) steuert, dadurch gekennzeichnet, daß die Aktivität des Genprodukts des Transkriptionsfaktorgenes abhängig ist von einem oder mehreren zellulären Regulatorproteinen, die spezifisch an dieses Genprodukt binden und dessen Aktivität beeinflussen.

2. Nukleinsäurekonstrukt gemäß Anspruch 1, dadurch gekennzeichnet, daß
- Komponente a): eine Aktivierungssequenz für die Transkription der Komponente b);
- Komponente b): ein Transkriptionsfaktor, enthaltend
b₁) eine Aktivierungsdomäne
b₂) eine Bindesequenz für ein zelluläres Regulatorprotein
b₃) eine DNA-Bindedomäne;
- Komponente c) eine Aktivierungssequenz, welche durch Bindung des Expressionsproduktes der Komponente b) aktiviert wird und die die Transkription der Komponente d) aktiviert; und
- Komponente d) ein Effektorgen
ist.

3. Nukleinsäurekontrukt nach Anspruch 1, dadurch gekennzeichnet, daß die Komponenten a, b und c in Form der Komponenten a', b' und c' ausgeführt sind:
- Komponente a'): eine Aktivierungssequenz für die Transkription der Komponente b') enthaltend
a₁) die DNA-Bindesequenz für ein zelluläres Regulatorprotein
a₂) einen basalen Promotor
- Komponente b') : einen Transkriptionsfaktor, der ein Repressorprotein darstellt und die Komponente c') hemmt
- Komponente c') eine Aktivierungssequenz, enthaltend
c₁) eine Aktivierungssequenz für die Transkription der Komponente d)
c₂) eine DNA-Sequenz, die das Repressorprotein [Komponente b')] bindet und hierdurch die Aktivierung der Transkription der Komponente d) inhibiert
- Komponente d) ein Effektorgen.

4. Nukleinsäurekonstrukt nach Anspruch 2, dadurch gekennzeichnet, daß die Komponente a) gleich ist der Komponente c).

5. Nukleinsäurekonstrukt nach Anspruch 2 oder 3, dadurch gekennzeichnet, daß die Komponente a) bzw. die Komponente c₁) eine unspezifisch, zellspezifisch, metabolisch spezifisch, virusspezifisch und/oder zellzyklusspezifisch aktivierbare Promotorsequenz darstellt.

6. Nukleinsäurekonstrukt nach Anspruch 5, dadurch gekennzeichnet, daß die Komponente a) oder die Komponente c₁) ausgewählt ist aus der Gruppe enthaltend
- Promotoren aktiviert in Endothelzellen, Peritonealzellen, Pleuralzellen, Epithelzellen der Haut, der Lunge, des Gastrointestinaltraktes, der Niere und der harnableitenden Wege, in Muskelzellen, in Bindegewebszellen, in blutbildenden Zellen, in Makrophagen, in Lymphozyten, in Leukämiezellen, in Tumorzellen oder in Gliazellen oder
- Promotorsequenzen von Viren, wie HBV, HCV, HSV, HPV, EBV, HTLV, CMV oder HIV
- Promotor- oder Enhancersequenzen aktiviert durch Hypoxie oder
- zellzyklusspezifische Aktivierungssequenzen der Gene für cdc25C, Cyclin A, cdc2, E2F-1, B-myb und DHFR
- Bindesequenzen für zellproliferationsabhängig auftretende oder aktivierte Transkriptionsfaktoren wie Monomere oder Multimere der Myc E-Box.

7. Nukleinsäurekonstrukt nach einem oder mehreren der Ansprüche 2 bis 6, dadurch charakterisiert, daß die Aktivierungsdomäne [(Komponente b1)] der Komponente b) ausgewählt ist aus der Gruppe enthaltend die Aktivierungsdomänen der Transkriptionsfaktoren Oct-2, Sp1, NFY, ITF-2, VP-16, c-Myc und CTF.

8. Nukleinsäurekonstrukte nach einem oder mehreren der Ansprüche 2 bis 7, dadurch charakterisiert, daß die Bindesequenz [Komponente b2) der Komponente b)] für ein zelluläres Regulatorprotein ein zelluläres Bindeprotein oder ein Teil dieses Bindeproteins ist.

9. Nukleinsäurekonstrukte nach Anspruch 8, dadurch charakterisiert, daß das zelluläre Bindeprotein oder ein Teil dieses Bindeproteins an ein zelluläres Regulatorprotein ausgewählt aus einer Gruppe enthaltend p53, pRb, p130, Max, MAD, VHL, cdk-4, MTS-1 (p16), WT-1, SMAD-2, DPC-4, bindet.

10. Nukleinsäurekonstrukt nach Anspruch 9, dadurch charakterisiert, daß die Komponente b') ausgewählt ist aus einer Gruppe von zellulären Bindeproteinen enthaltend
E2F-1, -2, -3, -4, -5, Cyclin-D₁, D₂, -D₃, oder -C, Cyclin A, - E, Myc, Transkriptionsfaktor PU.1 oder Elf-1, Elongin-B, -C, p14, p15, p16, p18, p21, p27, p53, Myc, cdk-4, DPC-4 und SMAD-2.

11. Nukleinsäurekonstrukt nach Anspruch 7, dadurch charakterisiert, daß die Bindesequenz [Komponente b2) der Komponente b)] für ein zelluläres Regulatorprotein ein virales Bindeprotein oder ein Teil dieses Bindeproteins ist.

12. Nukleinsäurekonstrukt nach Anspruch 11, dadurch charakterisiert, daß das virale Bindeprotein oder ein Teil dieses Bindeproteins an ein zelluläres Regulatorprotein ausgewählt aus einer Gruppe enthaltend p53, pRb (p110), NFKB, p130, CBF-1, Lyn-Tyrosinkinase, bak und bax bindet.

13. Nukleinsäurekonstrukt nach Anspruch 12, dadurch charakterisiert, daß die Komponente b2) ausgewählt ist aus einer Gruppe von viralen Bindeproteinen, enthaltend
IE 84 von CMV, E1B (55 Kd) von AV, EBNA-5 von EBV, BHFR von EBV, E6 von HPV-16 oder -18, x Protein von HBV, T-Antigen von SV40, E1A von AV, EBNA-2 von EBV, EBNA-1 von EBV, E7 von HPV, Tax von HIV, LMP-1 von EBV, LMP-2A oder LMP-2B von EBV, E1B (16 Kd) von AV, E1B (10 kD von AV.

14. Nukleinsäurekonstrukt nach Anspruch 7, dadurch charakterisiert, daß die Bindesequenz [Komponente b₂) der Komponente b)] für ein zelluläres Regulatorprotein ein Antikörper oder ein Teil dieses Antikörpers ist.

15. Nukleinsäurekonstrukt nach einem der vorhergehenden Ansprüche, dadurch charakterisiert, daß die Komponente c) mindestens eine DNA-Sequenz zur Bindung der Komponente b) enthält und durch diese Bindung die Expression von Komponente d) aktiviert wird.

16. Nukleinsäurekonstrukt nach Anspruch 15, bei welchem die DNA-Sequenz ausgewählt ist aus einer Gruppe enthaltend
die Bindesequenz (5'-CGGACAACTGTTGACCCG-3', SEQ ID NO.: 1), für das Gal4-Protein; die Bindesequenz (5'-TACTGTATGTACATACAGTA-3', SEQ ID NO.: 2) für das LexA-Protein; die Bindesequenz (5'-GAATTGTGAGCGCGCA CAATTC-3', SEQ ID NO.: 3) für das Lac I-Refpressorprotein; die Bindesequenz (5'-TCGAGTTTACCACTCCCTATCAGTGATAGAGAAAA GTGAAAG-3', SEQ ID NO.: 4) für das Tetracyclin-Repressorprotein und die Bindesequenz (5'-TAATGATGGGCG-3', SEQ ID NO.: 5) für das ZFHD-1 Protein.

17. Nukleinsäurekonstrukt nach einem oder mehreren der Ansprüche 11 bis 14, dadurch charakterisiert, daß die DNA-Bindesequenz für ein zelluläres Regulatorprotein [Komponente a₁)] eine DNA-Bindesequenz ist, ausgewählt aus der Gruppe enthaltend p53, W4-1, NF-Kappa B, E2F/DP oder Myc/Max Protein.

18. Nukleinsäurekonstrukt nach Anspruch 17, dadurch charakterisiert, daß der basale Promotor [Komponente a₂)] ausgewählt ist aus der Gruppe enthaltend die Promotoren von SV40, c-fos, U2 sn RNA und HSV-TK.

19. Nukleinsäurekonstrukt nach Anspruch 18, dadurch charakterisiert, daß der Repressor [Komponente b')] ausgewählt wird aus der Gruppe enthaltend das lac-Repressorgen oder das Tetrazyklinrepressorgen.

20. Nukleinsäurekonstrukt nach Anspruch 19, dadurch charakterisiert, daß die DNA-Bindesequenz für den Repressor [Komponente c')] mindestens eine lac-Operator-Bindesequenz oder mindestens eine Tetrazyklin-Operator-Bindesequenz enthält.

21. Nukleinsäurekonstrukt nach einem oder mehreren der Ansprüche 1 bis 20, dadurch gekennzeichnet, daß es sich bei dem Effektorgen (Komponente d) um ein Gen handelt, das für einen Wirkstoff kodiert, der ausgewählt ist aus der Gruppe enthaltend Cytokine, Chemokine, Wachstumsfaktoren, Rezeptoren für Cytokine, Chemokine oder Wachstumsfaktoren, antiproliferativ oder zytostatisch oder apoptotisch wirkende Proteine, Antikörper, Antikörperfragmente, Angiogeneseinhibitoren, Peptidhormone, Gerinnungsfaktoren, Gerinnungshemmer, fibrinolytische Proteine, auf den Blutkreislauf wirksame Peptide oder Proteine, Blutplasmaproteine und Antigene von Infektionserregem oder von Zellen oder von Tumoren, wobei das ausgewählte Antigen eine Immunreaktion bewirkt.

22. Nukleinsäurekonstrukt nach einem oder mehreren der Ansprüche 1 bis 20, dadurch gekennzeichnet, daß es sich bei dem Effektorgen um ein Gen handelt, das für ein Enzym kodiert, welches eine Vorstufe eines Pharmakons in ein Pharmakon spaltet.

23. Nukleinsäurekonstrukt nach einem oder mehreren der Ansprüche 1 bis 20, dadurch gekennzeichnet, daß es sich bei dem Effektorgen um ein Gen handelt, das für ein Ligand-Wirkstoff-Fusionsprotein oder ein Ligand-Enzyzm-Fusionsprotein kodiert, wobei der Ligand ausgewählt ist aus einer Gruppe enthaltend Cytokine, Wachstumsfaktoren, Antikörper, Antikörperfragmente, Peptidhormone, Mediatoren und Zelladhäsionsmoleküle.

24. Nukleinsäurekonstrukt nach Anspruch 2, dadurch gekennzeichnet, daß die Komponenten a), b), c) und d) wie folgt charakterisiert sind:
- Komponente a)
• der Promotor des cdc25C-Gens (Nukleinsäuren -290 bis +121)
- Komponente b)
• das nukleäre Lokalisationssignal (NLS) von SV40 (SV40 large T, Aminosäuren 126 bis 132; PKKKRKV (SEQ ID NO.: 13)
• die saure Transaktivierungsdomäne (TAD) von HSV-1 VP16 (Aminosäuren 406 bis 488)
• die RB-Bindesequenz des E2F-1 Proteins (Aminosäuren 409 bis 426 (LDYHFGLEEGEGIRDLFD) (SEQ ID NO.: 14)
• die cDNA für die DNA-Bindedomäne des Gal4-Proteins (Aminosäuren 1 bis 147)
- Komponente c)
• 10x die Bindesequenz für die Gal4 DNA-Bindesequenz mit der Nukleotidsequenz 5'-CGGACAATGTTGACCG-3' (SEQ ID NO.: 1)
• der basale Promotor von SV40 (Nukleinsäuren 48 bis 5191)
- Komponente d)
• die Sequenz GCCACC
• die cDNA für das Signalpeptid des Immunglobulins (Nukleotidsequenz 63 bis 107)
• die cDNA der β-Glucuronidase (Nukleotidsequenz 93 bis 1982).

25. Nukleinsäurekonstrukt nach Anspruch 2, dadurch gekennzeichnet, daß die Komponenten a), b), c) und d) wie folgt charakterisiert sind:
- Komponente a)
• der Promotor des cdc25C-Gens (Nukleinsäuren -290 bis +121)
- Komponente b)
• das nukleäre Lokalisationssignal (NLS) von SV40 (SV40 large T, Aminosäuren 126 bis 132; PKKKRKV (SEQ ID NO.: 13)
• die saure Transaktivierungsdomäne (TAD) von HSV-1 VP16 (Aminosäuren 406 bis 488)
• das E6-Protein des HPV-18 Virus (Nukleotidsequenz 100 bis 578)
• die cDNA für die DNA-Bindedomäne des Gal4-Proteins (Aminosäuren 1 bis 147)
- Komponente c)
• 10x die Bindesequenz für die Gal4 DNA-Bindesequenz mit der Nukleotidsequenz 5'-CGGACAATGTTGACCG-3' (SEQ ID NO.: 1)
• der basale Promotor von SV40 (Nukleinsäuren 48 bis 5191)
- Komponente d)
• die Sequenz GCCACC
• die cDNA für das Signalpeptid des Immunglobulins (Nukleotidsequenz 63 bis 107)
• die cDNA der β-Glucuronidase (Nukleotidsequenz 93 bis 1982).

26. Nukleinsäurekonstrukt nach einem der vorhergehenden Ansprüche dadurch gekennzeichnet, daß es sich bei der Nukleinsäure um DNS handelt.

27. Nukleinsäurekonstrukt nach einem der vorhergehenden Ansprüche dadurch gekennzeichnet, daß das Nukleinsäurekonstrukt eingefügt ist in einen Vektor.

28. Nukleinsäurekonstrukt nach Anspruch 27, dadurch gekennzeichnet, daß es sich um einen Plasmidvektor handelt.

29. Nukleinsäurekonstrukt nach Anspruch 27, dadurch gekennzeichnet, daß es sich um einen viralen Vektor handelt.

30. Nukleinsäurekonstrukt nach einem oder mehreren der Ansprüche 1 bis 29, dadurch gekennzeichnet, daß es äußerlich, peroral, intravesikal, nasal, intrabronchial oder in den Magen-Darm-Trakt verabreicht oder in ein Organ, in eine Körperhöhle, in die Muskulatur, subkutan oder in den Blutkreislauf injiziert wird zur Prophylaxe oder Therapie einer Erkrankung.

31. Isolierte Zelle dadurch gekennzeichnet, daß sie ein Nukleinsäurekonstrukt gemäß einem der Ansprüche 1-29 enthält.

32. Verwendung eines Nukleinsäurekonstruktes nach einem oder mehreren der Ansprüche 1 bis 29 oder einer Zelle nach Anspruch 31 zur Herstellung eines Heilmittels zur Behandlung einer Erkrankung ausgewählt aus der Gruppe enthaltend Infektionen, Tumore, Leukämien, Autoimmunerkrankungen, Allergien, Arthritiden, Entzündungen, Organabstoßungen, Transplantat gegen WirtReaktionen, Blutgerinnungserkrankungen, Kreislauferkrankungen, Blutarmut, Hormonerkrankungen und ZNS-Schäden.

33. Verfahren zur Herstellung der Nukleinsäurekonstrukte nach einem oder mehreren der Ansprüche 1 bis 30, bei dem die einzelnen Elemente schrittweise zusammenligiert werden.

34. Verwendung einer Zelle nach Anspruch 31 zur Herstellung eines Heilmittels zur Prophylaxe oder Therapie von Erkrankungen gemäß Anspruch 32 dadurch gekennzeichnet, daß mindestens eine Zelle äußerlich, intravesikal, nasal, intrabronchial, oral oder in den Magen-Darm-Trakt verabreicht oder in ein Organ, in eine Körperhöhle, in die Muskulatur, subkutan oder in den Blutkreislauf injiziert wird.


SEQUENZPROTOKOLL

(1) ALGEMEINE INFORMATION:

(i) ANMELDER:
(A) NAME: Hoechst Marion Roussel Deutschland GmbH
(B) STRASSE: -
(C) ORT: Frankfurt
(D) BUNDESLAND: -
(E) LAND: Deutschland
(F) POSTLEITZAHL: -
(G) TELEPHON: 069-305-3005
(H) TELEFAX: ----
(I) TELEX: -

(ii) ANMELDETITEL: Onkogen- oder virusgesteuerte
Expressionssysteme

(iii) ANZAHL DER SEQUENZEN: 14

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..17

(xi) SEQUENZBESCHREIBUNG: SEQ ID NO: 1:

CGGACAACTG TTGACCG 17

(2) INFORMATION ZU SEQ ID NO: 2:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 2:

TACTGTATGT ACATACAGTA 20

(2) INFORMATION ZU SEQ ID NO: 3:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 3:

GAATTGTGAG CGCTCACAAT TC 22

(2) INFORMATION ZU SEQ ID NO: 4:

(i) SEQUENZ CHARAKTERISTIKA:
(A) LÄNGE: 42 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..42

(xi) SEQUENZBESCHREIBUNG: SEQ ID NO: 4:

TCGAGTTTAC CACTCCCTAT CAGTGATAGA GAAAAGTGAA AG 42

(2) INFORMATION ZU SEQ ID NO: 5:

(i) SEQUENZ CHARAKTERISTIKA:
(A) LÄNGE: 11 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..11

(xi) SEQUENZBESCHREIBUNG: SEQ ID NO: 5:

TAATGATGGC G 11

(2) INFORMATION ZU SEQ ID NO: 6:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 6:

ATAATTGGGC AAGTCTAGGA A 21

(2) INFORMATION ZU SEQ ID NO: 7:

(i) SEQUENZ CHARAKTERISTIKA:
(A) LÄNGE: 10 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..10

(xi) SEQUENZBESCHREIBUNG: SEQ ID NO: 7:

GGGACTTTCC 10

(2) INFORMATION ZU SEQ ID NO: 8:

(i) SEQUENZ CHARAKTERISTIKA:
(A) LÄNGE: 14 Basenpaare
(B) ART: Nukleinsäure
(C) STRANGFORM: Einzel

(D) TOPOLOGIE: linear

(ii) ART DES MOLEKÜLS: DNS (genomisch)

(ix) MERKMALE:
(A) NAME/SCHLÜSSEL: exon
(B) LAGE: 1..14

(xi) SEQUENZBESCHREIBUNG: SEQ ID NO: 8:

TTTTCCCGCC AAAA 14

(2) INFORMATION ZU SEQ ID NO: 9:

(i) SEQUENZ CHARAKTERISTIKA:
(A) LÄNGE: 16 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..16

(xi) SEQUENZBESCHREIBUNG: SEQ ID NO: 9:

TTTTCCCGCC TTTTTT 16

(2) INFORMATION ZU SEQ ID NO: 10:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..17

(xi) SEQUENZBESCHREIBUNG: SEQ ID NO: 10:

TTTTCCCGCG CTTTTTT 17

(2) INFORMATION ZU SEQ ID NO: 11:

(i) SEQUENZ CHARAKTERISTIKA:
(A) LÄNGE: 26 Basenpaare
(B) ART: Nukleinsäure

(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..26

(xi) SEQUENZBESCHREIBUNG: SEQ ID NO: 11:

GGAAGCAGAC CACGTGGTCT GCTTCC 26

(2) INFORMATION ZU SEQ ID NO: 12:

(i) SEQUENZ CHARAKTERISTIKA:
(A) LÄNGE: 41 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..41

(xi) SEQUENZBESCHREIBUNG: SEQ ID NO: 12:

GGCCGATGGG CAGATAGAGG GGGCCGATGG GCAGATAGAG G 41

(2) INFORMATION ZU SEQ ID NO: 13:

(i) SEQUENZ CHARAKTERISTIKA:
(A) LÄNGE: 7 Aminosäuren
(B) ART: Peptid
(C) STRANGFORM: Einzel
(D) TOPOLOGIE: linear

(ii) ART DES MOLEKÜLS: Peptid

(ix) MERKMALE:
(A) NAME/SCHLÜSSEL: Peptide
(B) LAGE: 1..7

(xi) SEQUENZBESCHREIBUNG: SEQ ID NO: 13:

Pro Lys Lys Lys Arg Lys Val
1               5

(2) INFORMATION ZU SEQ ID NO: 14:

(i) SEQUENZ CHARAKTERISTIKA:
(A) LÄNGE: 18 Aminosäuren
(B) ART: Peptid
(C) STRANGFORM: Einzel
(D) TOPOLOGIE: linear

(ii) ART DES MOLEKÜLS: Peptid

(ix) MERKMALE:
(A) NAME/SCHLÜSSEL: Peptide
(B) LAGE: 1..18

(xi) SEQUENZBESCHREIBUNG: SEQ ID NO: 14:

Leu Asp Tyr His Phe Gly Leu Glu Glu Gly Glu Gly Ile Arg Asp Leu
1               5                   10                  15

Phe Asp